# FASCICULE DE BREVET EUROPEEN

(11) **EP 0 769 967 B1**
(45) Date de publication et mention de la délivrance du brevet: **12.12.2007**
(21) Numéro de dépôt: 95928905.9
(22) Date de dépôt: 21.08.1995
(51) Int. Cl.: A61K 47/48, A61P 35/00

(54) **CONJUGUES COMPRENANT UN AGENT ANTITUMORAL ET LEUR UTILISATION**
KONJUGATE ENTHALTEND EIN ANTITUMORALES MITTEL UND DEREN VERWENDUNG
CONJUGATES COMPRISING AN ANTITUMOUR AGENT AND THEIR USE

(30) Priorité: 19.08.1994 BE 9400752; 19.08.1994 BE 9400751
(43) Date de publication de la demande: 02.05.1997
(60) Demande divisionnaire: 07119790.9
(73) Titulaire: LA REGION WALLONNE, B-1050 Bruxelles (BE); UNIVERSITE CATHOLIQUE DE LOUVAIN, 1348 Louvain-la-Neuve (BE)
(72) Inventeur: TROUET, André, B-3020 Herent (BE); BAURAIN, Roger, B-1970 Wezembeek-Oppem (BE)
(74) Mandataire: Brants, Johan P.E.
(86) Numéro de dépôt international: PCT/BE1995/000076
(87) Numéro de publication internationale: WO 1996/005863

(56) Documents cités:
- EP-A- 0 041 935
- EP-A- 0 044 090
- EP-A- 0 126 685
- EP-A- 0 208 615
- BE-A- 869 485
- BE-A- 882 541
- DATABASE CHEMABS CHEMICAL ABSTRACTS SERVICE, COLUMBUS, OHIO, US AN=97:150636, BAURAIN, R. ET AL 'Antitumor activity of daunorubicin linked to proteins: lysosomal hydrolysis and antitumor activity of conjugates prepared with peptidic spacer arms' & CURR. CHEMOTHER. IMMUNOTHER., PROC. INT. CONGR. CHEMOTHER., 12TH (1982), MEETING DATE 1981, VOLUME 2, 1430-2. EDITOR(S): PERITI, PIERO;GIALDRONI GRASSI, GIULIANA. PUBLISHER: AM. SOC. MICROBIOL., WASHINGTON, D. C. CODEN: 48HGAR,
- JOURNAL OF MEDICINAL CHEMISTRY, vol. 23, WASHINGTON US, pages 1171-1174, R. BAURAIN ET AL. 'AMINO ACID AND DIPEPTIDE DERIVATIVES OF DAUNORUBICIN. 2. CELLULAR PHARMACOLOGY AND ANTITUMOR ACTIVITY ON L1210 LEUKEMIC CELLS IN VITRO AND IN VIVO.'
- J. CONTROLLED RELEASE (1994), 31(1), 89-97 CODEN: JCREEC;ISSN: 0168-3659, MARRE, ANNE DE ET AL 'Evaluation of the hydrolytic and enzymic stability of macromolecular Mitomycin C derivatives'
- JOURNAL OF MEDICINAL CHEMISTRY, vol. 23, WASHINGTON US, pages 1166-1170, M. MASQUELIER ET AL. 'AMINO ACIDS AND DIPEPTIDE DERIVATIVES OF DAUNORUBICIN. 1. SYNTHESIS, PHYSICOCHEMICAL PROPERTIES , AND LYSOSOMAL DIGESTION.'
- CHEMICAL ABSTRACTS, vol. 97, no. 18, 1 Novembre 1982 Columbus, Ohio, US; abstract no. 150635, MASQUELIER, M. ET AL 'Antitumor activity of daunorubicin linked to proteins: biological and antitumor properties of peptidic derivatives of daunorubicin used as intermediates' & CURR. CHEMOTHER. IMMUNOTHER., PROC. INT. CONGR. CHEMOTHER., 12TH (1982), MEETING DATE 1981, VOLUME 2, 1428-30. EDITOR(S): PERITI, PIERO;GIALDRONI GRASSI, GIULIANA. PUBLISHER: AM. SOC. MICROBIOL., WASHINGTON, D. C. CODEN: 48HGAR,
- CHEMICAL ABSTRACTS, vol. 121, no. 7, 15 Août 1994 Columbus, Ohio, US; abstract no. 79924, KENNETT, C. N. ET AL 'Comparative histochemical, biochemical and immunocytochemical studies of cathepsin B in human gingiva' & J. PERIODONTAL RES. (1994), 29(3), 203-13 CODEN: JPDRAY;ISSN: 0022-3484,
- TETRAHEDRON LETT. (1995), 36(9), 1413-16 CODEN: TELEAY;ISSN: 0040-4039, SEITZ, DAVID E. ET AL 'Synthesis and chemical properties of a series of doxorubicin enaminomalonyl-. beta.- alanine derivatives'
- CHEMICAL ABSTRACTS, vol. 107, no. 15, 12 Octobre 1987 Columbus, Ohio, US; abstract no. 127965, WHALLEY, E. T. 'Receptors mediating the increase in vascular permeability to kinins: comparative studies in rat, guinea pig and rabbit' & NAUNYN-SCHMIEDEBERG'S ARCH. PHARMACOL. (1987), 336(1), 99-104 CODEN: NSAPCC;ISSN: 0028-1298,

## Description

### Objet de l'invention.

La présente invention concerne des nouveaux composés, la composition pharmaceutique les comprenant et l'utilisation de ceux-ci pour la préparation de médicaments destinés au traitement de tumeurs cancéreuses.

### Etat de la technique et arrière-plan technologique à la base de l'invention.

De nombreux agents tumoraux, tels que les anthracyclines et les alcaloïdes vinca, ont été développés ces dernières années et sont particulièrement efficaces pour le traitement des cancers. Cependant, ces molécules sont souvent caractérisées in vivo par une toxicité aiguë, en particulier une toxicité médullaire et muqueuse ainsi qu'une toxicité chronique cardiaque chez les anthracyclines et neurologique chez les alcaloïdes vinca.

Aussi, on a cherché à développer des agents antitumoraux plus spécifiques, de manière à ce qu'ils soient plus efficaces contre les cellules tumorales, et par conséquent, à diminuer les effets secondaires de ces produits (toxicité, destruction de cellules non tumorales, ...).

Le brevet US-4,296,105 décrit des dérivés de doxorubicine liés à un acide aminé, éventuellement substitué, qui possèdent in vitro une activité antitumorale plus élevée et une toxicité plus faible que la doxorubicine.

Cependant, comme ces dérivés ont un site d'action intracellulaire, ces molécules sont encore susceptibles de pénétrer dans les cellules tumorales et dans les cellules normales.

Aussi, ces dernières années, de nouveaux agents thérapeutiques ont été proposés sous forme de prodrogues.

Les prodrogues sont des molécules susceptibles de se transformer en drogues (composés thérapeutiques actifs) par certaines modifications chimiques ou enzymatiques de leur structure.

Cependant, ces prodrogues sont également caractérisées par une faible stabilité dans le sang et le sérum, qui comportent des enzymes qui inactivent ces molécules.

Les documents Chemical Abstract 97:150635, Journal of Medicinal Chemistry, Vol. 23, pp. 1171-1174 (1980); Drugs Exp. Clin., Vol. 9, pp. 303-311 (1983); Journal of Medical Chemistry, Vol. 23, pp. 1166-1170 (1980) et la demande de brevet BE-882.541 décrivent des prodrogues comprenant un vecteur lié par un bras peptidique à la drogue. De préférence, le bras est constitué de quatre amino-acides et est lié par sa fonction carboxylique libre à la fonction amine libre de dérivés d'anthracyclines telles que la daunorubicine.

En outre, le bras de ces prodrogues est lié par sa fonction amine libre à un vecteur constitué d'une macromolécule (protéine telle que la BSA, des immunoglobulines, ...) qui permet l'endocytose sélective de la prodrogue par des cellules cibles.

Il est également connu que le méthotrexate peut être utilisé pour le traitement de réactions inflammatoires telles que les rhumatismes, mais sa toxicité élevée limite ses applications.

### Buts de l'invention.

La présente invention vise à fournir de nouveaux composés comprenant un agent thérapeutique antitumoral, en particulier des prodrogues comprenant un agent thérapeutique antitumoral, présentant des propriétés thérapeutiques améliorées par rapport aux produits de l'état de la technique, en particulier des propriétés thérapeutiques améliorées dans le traitement des tumeurs cancéreuses.

Un but particulier de la présente invention vise à obtenir des prodrogues qui présentent une spécificité d'action élevée, une toxicité réduite et une stabilité améliorée dans le sérum et le sang.

### Eléments caractéristiques de la présente invention.

La présente invention concerne des composés (W-Z-M) tels que définis à la revendication 1. Ces composés comprennent un élément choisi parmi le groupe constitué par les agents thérapeutiques antitumoraux, possédant un site actif intracellulaire (S.A.), lié à un ligand (W-Z) comprenant un bras (Z) lié à un groupement terminal (W) dans lequel la liaison entre le bras (Z) du ligand (W-Z) et l'élément (M) empêche la pénétration cellulaire du composé (W-Z-M) ; dans lequel cette liaison est clivable sélectivement par des facteurs sécrétés par des cellules cibles, de manière à permettre la pénétration de l'agent thérapeutique (M) dans lesdites cellules cibles, et dans lequel le groupement terminal (W) assure la stabilité du composé (W-Z-M) dans le sérum et dans le sang circulant, caractérisé en ce que la liaison entre le groupement terminal (W) et le bras (Z) ainsi que la liaison entre le bras (Z) et l'élément (M) sont des liaisons covalentes n'affectant pas les propriétés du composés (W-Z-M), en ce que le bras (Z) du ligand (W-Z) est un peptide constitué d'au moins deux acides aminés éventuellement substitués, en ce que le groupement (W) est la β-alanine de Formule H₂N-CH₂-CH₂-CO₂H, et en ce que la liaison entre l'élément (M) et le bras (Z) du ligand (W-Z) est un lien peptidique.

On entend par facteurs sécrétés par des cellules cibles, en particulier par des cellules tumorales, des enzymes telles que des protéases ou peptidases, sécrétées de manière spécifique dans le milieu extracellulaire par lesdites cellules.

Ces enzymes sont donc susceptibles de cliver sélectivement la liaison existant entre l'élément (M) et le bras (Z) du ligand (W-Z) de manière à permettre la pénétration de l'agent thérapeutique, préférentiellement dans lesdites cellules, et d'assurer de cette manière leur destruction et/ou de bloquer leur prolifération.

On entend par groupement terminal (W) assurant la stabilité du composé selon l'invention dans le sérum et dans le sang circulant, la β-alanine, qui réduit ou inhibe le clivage du composé selon l'invention dans le sérum et le sang circulant, en particulier qui réduit ou inhibe l'hydrolyse du composé selon l'invention par des protéinases et peptidases présentes dans le sérum et/ou le sang circulant, en particulier les peptidases et protéinases associées aux globules rouges.

En particulier, un groupement terminal (W) assure la stabilité du composé selon l'invention lorsque moins de 20%, de préférence moins de 2%, du composé est clivé par lesdites enzymes pendant sa conservation dans du sang humain à 37°C pendant plus de 2 heures.

Selon l'invention, le groupement (W) est la β-alanine de formule : NH₂-CH₂-CH₂-COOH, lié par sa fonction carboxylique au bras (Z).

Selon l'invention, la liaison entre l'élément (M) et le bras (Z) du ligand (W-Z) consiste en un lien peptidique et le bras (Z) est un peptide constitué d'au moins deux acides aminés éventuellement substitués.

Le bras (Z) du ligand (W-Z) est constitué de préférence par la succession des acides aminés suivants : L-Leucyl-L-Alanyl-L-Leucyl, L-Leucyl-L-Alanyl ou L-Alanyl-L-Leucyl-L-Phénylalanyl ou L-Alanyl-L-Leucyl, liés par leur fonction carboxylique à l'élément (M).

Selon l'invention, l'agent thérapeutique (M) est un agent thérapeutique utilisé en chimiothérapie du cancer, de préférence choisi parmi les agents thérapeutiques décrits par Bruce A. Chabner et Jerry M. Collins (Cancer Chemotherapy, Lippincott Ed., ISBN 0-397-50900-6 (1990)), et susceptible de se fixer au ligand (W-Z) par un lien peptidique au groupement (Z) du ligand (W-Z).

En particulier, l'agent thérapeutique est choisi parmi le groupe constitué par les anthracyclines, les dérivés d'acide folique, les alcaloïdes vinca, le mitoxanthrone, la calichéamycine, le cytosine arabinoside (ARA-C), l'adénosine arabinoside (ARA-A), le fludarabine phosphate, le melphalan, la bléomycine, la mitomycine, la L-canavanine, les taxoïdes, la camptothécine et leurs dérivés, en particulier le TOPOTECAN ® (hydrochlorure de 9-diméthylaminométhyle-10-hydroxy camptothécine), éventuellement liés à un acide aminé substitué ou non. La substitution sur l'acide aminé peut être toute substitution n'affectant pas les propriétés du composé (W-Z-M) selon l'invention.

On entend par dérivés de ces molécules, lesdites molécules modifiées par un groupement chimique permettant leur fixation au bras (Z) du ligand (W-Z) par un lien covalent qui n'affecte pas l'activité thérapeutique de la molécule originale.

De préférence, dans le composé selon l'invention, l'agent thérapeutique (M) est choisi parmi le groupe des anthracyclines, en particulier la doxorubicine (DOX) et la daunorubicine (DNR), éventuellement liées à un acide aminé substitué ou non.

Avantageusement, l'agent thérapeutique correspond à la formule générale : 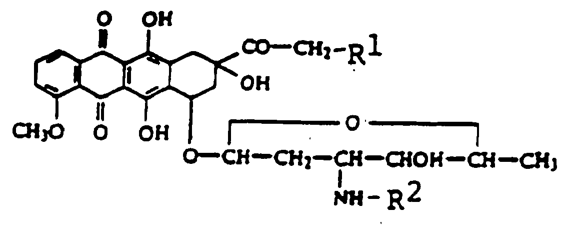 dans laquelle :
- R¹ est un atome d'hydrogène ou un groupement OH,
- R² est un atome d'hydrogène ou un radical de formule 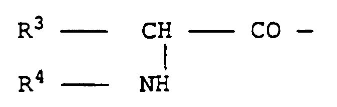 dans laquelle :
   - R³ est un atome d'hydrogène ou un radical alkyle, éventuellement substitué,
   - R⁴ représente un atome d'hydrogène ou forme avec R³ un radical alkylène comportant 3 ou 4 atomes de carbone.

De préférence, R² est un radical de formule : 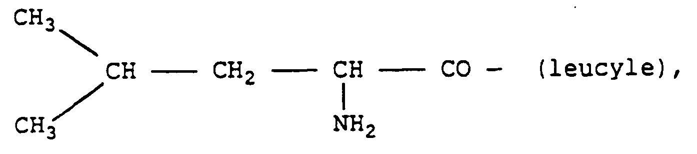 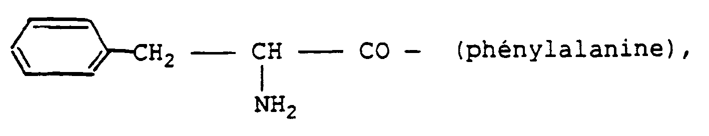 ou un de leurs isomères.

Selon une forme d'exécution préférée de l'invention, le composé est la βAlanyl-L-Leucyl-L-Alanyl-L-Leucyl-daunorubicine (identifiée par βAla-Leu-Ala-Leu-DNR partout ci-dessous), la βAlanyl-L-Leucyl-L-Alanyl-L-Leucyl-doxorubicine (identifiée par βAla-Leu-Ala-Leu-DOX partout ci-dessous), la βAlanyl-L-Alanyl-L-Leucyl-L-Phénylalanyl-daunorubicine (identifiée par ZAla-Ala-Leu-Phé-DNR partout ci-dessous) ou la βAlanyl-L-Alanyl-L-Leucyl-L-Phénylalanyl-doxorubicine (identifiée par βAla-Ala-Leu-Phé-DOX partout ci-dessous).

La présente invention concerne également la composition pharmaceutique comprenant le composé selon l'invention et éventuellement un adjuvant ou véhicule pharmaceutique acceptable.

Ces compositions peuvent par exemple être administrées par voie parentérale ou intraveineuse. Les compositions selon l'invention pour administration par voie parentérale peuvent être notamment des solutions stériles aqueuses ou non aqueuses, des suspensions ou des émulsions. Comme solvant ou véhicule pharmaceutique acceptable, on peut employer le propylène-glycol, le polyéthylène-glycol, des esters organiques injectables, par exemple l'oléate d'éthyle, ou les cyclodextrines. Ces compositions peuvent également comprendre des agents mouillants, émulsifiants et/ou dispersants.

La stérilisation peut se faire de plusieurs façons, par exemple à l'aide d'un filtre bactériologique en incorporant à la composition des agents stérilisants, ou par irradiation. Elles peuvent également être préparées sous forme de compositions solides stériles, qui peuvent être dissoutes au moment de l'emploi dans de l'eau stérile ou tout autre milieu stérile injectable.

La présente invention peut également comprendre des adjuvants bien connus de l'Homme du Métier (vitamine C, agents antioxydants, ...) pouvant être utilisés en synergie avec le composé selon l'invention pour améliorer et prolonger le traitement des tumeurs cancéreuses.

Les doses d'administration minimales des composés selon l'invention à un patient, sont les doses habituelles des agents thérapeutiques antitumoraux susmentionnés, telles que notamment décrites par Bruce A. Chabner et Jerry M. Collins (Cancer Chemotherapy, Lippincott Ed., ISBN 0-397-50900-6 (1990)) ou des doses d'administration plus élevées.

Les doses administrées varient donc en fonction de l'agent thérapeutique utilisé pour la préparation du composé selon l'invention.

La présente invention concerne l'utilisation de la composition pharmaceutique selon l'invention pour la préparation d'un médicament destiné au traitement des tumeurs cancéreuses, consistant en l'administration, en particulier par voie parentérale ou intraveineuse, à un patient de la composition pharmaceutique selon l'invention.

### Brève description des figures.

- La figure 1: représente le mécanisme d'action du composé selon l'invention sur son site d'action (S.A.) dans une cellule cible (C.C.).
- La figure 2: représente le mécanisme d'action du composé selon l'invention dans une cellule normale (C.N.).
- La figure 3: représente l'hydrolyse de la β-Ala-Leu-Ala-Leu-daunorubicine dans du milieu conditionné de cellules de carcinome mammaire humain MCF7/6 (chromatogramme au temps initial (a) et après deux heures d'incubation (b)).
- La figure 4: représente l'hydrolyse de la β-Ala-Leu-Ala-Leu-daunorubicine dans du milieu conditionné de cellules de carcinome mammaire humain MCF7/ADR (chromatogramme au temps initial (a) et après une heure d'incubation (b)).
- La figure 5: représente l'hydrolyse de la β-Ala-Leu-Ala-Leu-daunorubicine dans du milieu conditionné de cellules de carcinome du colon HT29 (chromatogramme au temps initial (a) et après deux heures d'incubation (b)).
- La figure 6: représente l'accumulation de la daunorubicine (DNR) à la concentration de 10 µg/ml par des cellules MCF7/6 confluentes.
- La figure 7: représente l'accumulation de la N-L-Leucyl-daunorubicine (Leu-DNR) à la concentration de 10 µg eq. DNR/ml par des cellules MCF7/6 confluentes.
- La figure 8: représente l'accumulation de la β-Ala-Leu-Ala-Leu-daunorubicine à la concentration de 10 µg eq. DNR/ml par des cellules MCF7/6 confluentes.
- La figure 9: représente l'accumulation de la daunorubicine (DNR) à la concentration de 10 µg/ml par des cellules fibroblastiques MRC5 confluentes.
- La figure 10: représente l'accumulation de la N-L-Leucyl-daunorubicine (Leu-DNR) à la concentration de 10 µg eq. DNR/ml par des cellules fibroblastiques MRC5 confluentes.
- La figure 11: représente l'accumulation de la β-Ala-Leu-Ala-Leu-daunorubicine à la concentration de 10 µg eq. DNR/ml par des cellules fibroblastiques MRC5 confluentes.
- La figure 12: représente la cytotoxicité de la daunorubicine (DNR), la L-Leu-daunorubicine et la β-Ala-Leu-Ala-Leu-daunorubicine vis-à-vis des cellules MCF7/6 maintenues en croissance pendant 72 heures en présence des anthracyclines.
- La figure 13: représente la cytotoxicité de la daunorubicine (DNR), la L-Leu-daunorubicine et la β-Ala-Leu-Ala-Leu-daunorubicine vis-à-vis des cellules fibroblastiques MRC5 maintenues en croissance pendant 72 heures en présence des anthracyclines.
- La figure 14: représente la mortalité des souris NMRI femelles après administration i.p. 5 jours consécutifs de la daunorubicine (DNR) aux doses totales comprises entre 2.0 et 3.5 mg/kg.
- La figure 15: représente l'évolution du poids moyen des souris NMRI femelles ayant reçu par voie i.p. 5 jours consécutifs des doses totales de daunorubicine comprises entre 2.0 et 3.5 mg/kg. Les poids sont exprimés en pourcentage du poids initial moyen pour chaque groupe.
- La figure 16: représente la mortalité des souris NMRI femelles après administration i.p. 5 jours consécutifs de la β-Ala-Leu-Ala-Leu-daunorubicine à des doses totales comprises entre 10 et 60 mg/kg.
- La figure 17: représente l'évolution du poids moyen des souris NMRI femelles ayant reçu par voie i.p. 5 jours consécutifs des doses totales de β-Ala-Leu-Ala-Leu-daunorubicine comprises entre 10 et 60 mg/kg. Les poids sont exprimés en pourcentage du poids initial moyen pour chaque groupe.
- La figure 18: représente la mortalité des souris NMRI femelles après administration i.v. unique de la daunorubicine (DNR) à des doses comprises entre 10 et 35 mg/kg.
- La figure 19: représente l'évolution du poids moyen des souris NMRI femelles ayant reçu par voie i.v. des doses de daunorubicine (DNR) comprises entre 10 et 35 mg/kg. Les poids sont exprimés en pourcentage du poids initial moyen pour chaque groupe.
- La figure 20: représente l'évolution du poids moyen des souris NMRI femelles ayant reçu par voie i.v. des doses de β-Ala-Leu-Ala-Leu-daunorubicine comprises de 30 et 60 mg/kg. Les poids sont exprimés en pourcentage du poids initial moyen pour chaque groupe.
- La figure 21: représente l'évolution du poids moyen des souris NMRI femelles ayant reçu par voie i.v. soit une dose de 30 mg/kg de β-Ala-Leu-Ala-Leu-daunorubicine, soit deux doses de 30 mg/kg chacune deux jours consécutifs. Les poids sont exprimés en pourcentage du poids initial moyen pour chaque groupe.
- La figure 22: représente la cinétique d'hydrolyse enzymatique de la β-Ala-Leu-Ala-Leu-doxorubicine en L-Ala-L-Leu-doxorubicine et L-Leu-doxorubicine dans un milieu conditionné par des cellules MCF7/6.
- La figure 23: représente l'accumulation de la doxorubicine (DOX) à la concentration de 10 µg/ml par des cellules MCF7/6 confluentes.
- La figure 24: représente l'accumulation de la L-Leu-doxorubicine à la concentration de 10 µg/ml par des cellules MCF7/6 confluentes.
- La figure 25: représente l'accumulation de la β-Ala-Leu-Ala-Leu-doxorubicine à la concentration de 10 µg/ml par des cellules MCF7/6 confluentes.
- La figure 26: représente l'accumulation de la doxorubicine (DOX) à la concentration de 10 µg/ml par des cellules fibroblastiques MRC5 confluentes.
- La figure 27: représente l'accumulation de la L-Leu-doxorubicine à la concentration de 10 µg/ml par des cellules fibroblastiques MRC5 confluentes.
- La figure 28: représente l'accumulation de la β-Ala-Leu-Ala-Leu-doxorubicine à la concentration de 10 µg/ml par des cellules fibroblastiques MRC5 confluentes.
- La figure 29: représente la cytotoxicité de la doxorubicine (DOX), de la L-Leu-doxorubicine et de la β-Ala-Leu-Ala-Leu-doxorubicine vis-à-vis des cellules MCF7/6 maintenues en croissance pendant 72 heures en présence desdits dérivés d'anthracycline.
- La figure 30: représente la cytotoxicité de la doxorubicine (DOX), de la L-Leu-doxorubicine et de la β-Ala-Leu-Ala-Leu-doxorubicine vis-à-vis des cellules fibroblastiques MRC5 maintenues en croissance pendant 72 heures en présence desdits dérivés d'anthracycline.
- La figure 31: représente la variation de la masse tumorale moyenne d'une tumeur mammaire humaine MCF7/6 implantée chez des souris athymiques au jour T-21 en fonction de l'administration de doxorubicine (DOX) et de β-Ala-Leu-Ala-Leu-doxorubicine (super DOX).
- La figure 32: représente la variation du poids moyen de souris (grammes) traitées par l'administration de doxorubicine (DOX) et de β-Ala-Leu-Ala-Leu-doxorubicine (super DOX).

### Description d'une forme d'exécution préférée de l'invention

La présente invention repose sur la découverte inattendue qu'il est possible de rendre un agent thérapeutique (M) antitumoral, inactif par la liaison avec un ligand (W-Z) qui empêche la pénétration intracellulaire de l'agent thérapeutique (M) dans des cellules normales (C.N.) et des cellules cibles (C.C.).

Selon l'invention, lesdites cellules cibles sont des cellules tumorales.

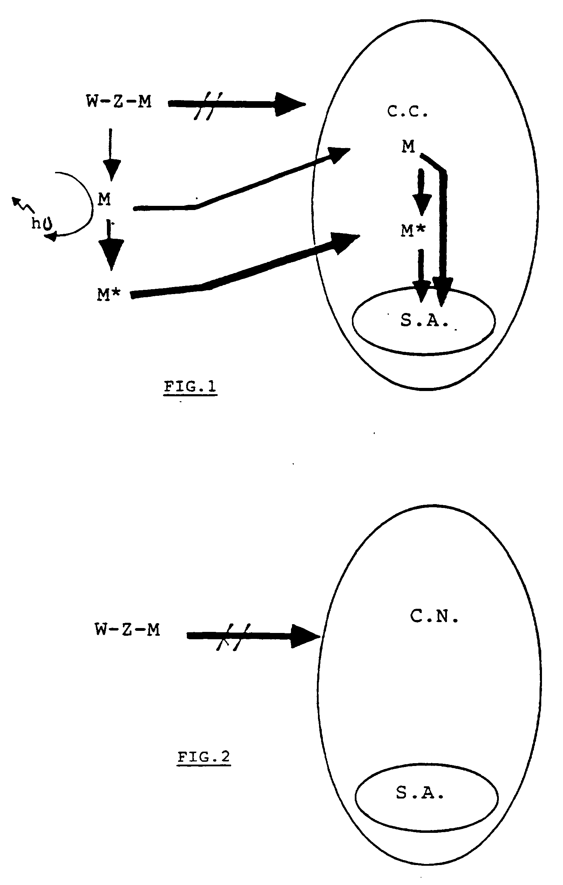

De manière inattendue, les cellules cibles (C.C.) libèrent dans le milieu extracellulaire des enzymes telles que des protéases ou des peptidases qui sont susceptibles d'hydrolyser sélectivement une liaison covalente entre le bras (Z) du ligand (W-Z) et l'agent thérapeutique (M), de manière à permettre la pénétration de l'agent thérapeutique (M) dans lesdites cellules cibles (C.C.). Dans la cellule cible, l'agent thérapeutique (M) agit soit directement sur son site d'action intracellulaire spécifique (S.A.) ou après une modification sous l'action de protéases intracellulaires, se modifie en un autre agent thérapeutique (M*) et tue la cellule cible (C.C) ou bloque sa prolifération (figure 1).

Comme les cellules normales ne libèrent pas ou peu lesdites enzymes in vivo, le composé selon l'invention est maintenu inactif et ne pénètre pas dans les cellules normales (figure 2).

Le composé décrit dans la présente demande de brevet répond à ce mécanisme d'action, car il remplit les critères nécessaires pour être administré de manière efficace :
1. L'agent thérapeutique antitumoral (M) :
   - possède un site d'action intracellulaire,
   - une haute activité spécifique,
   - un groupement chimique permettant une liaison covalente avec un ligand (W-Z);
   - si le groupement chimique requis n'est pas essentiel à l'activité de l'agent thérapeutique (M), celui-ci ne doit pas être restitué intact après hydrolyse enzymatique de ladite liaison covalente.
2. La liaison avec le ligand (W-Z) empêche la pénétration intracellulaire de l'agent thérapeutique (M) tant dans les cellules normales que dans les cellules cibles.
3. Le composé selon l'invention reste stable dans le sérum et dans le sang, et est insensible à l'action des protéinases et peptidases circulantes et associées aux globules rouges.
4. La liaison covalente existant entre l'agent thérapeutique antitumoral (M) et le ligand (W-Z) est dégradée partiellement ou totalement par les enzymes sécrétées par les cellules cibles.
5. La nature du ligand (W-Z) et sa liaison à l'agent thérapeutique (M) sont déterminées en fonction des enzymes sécrétées par les cellules cibles.
6. Le composé est moins toxique in vivo que l'agent thérapeutique de départ. Cette diminution de toxicité concerne notamment les effets aigus tels que la toxicité médullaire et mucosale, ainsi que la toxicité cardiaque ou neurologique éventuelle.

### Dérivés d'anthracyclines.

L'agent thérapeutique (M) selon l'invention peut être un dérivé d'anthracycline, en particulier la doxorubicine, la daunorubicine, le 4-épi-doxorubicine, le 4-déméthoxy-doxorubicine (Idarubicine), le 4'-tetrahydropyranyl-doxorubicine (Pirarubicine), la carminomycine, l'ésorubicine et la 4'-iodo-doxorubicine.

Le ligand (W-Z) est ensuite lié par sa fonction carboxylique à l'extrémité α-amino de l'agent thérapeutique.

### Dérivés d'acide folique.

L'agent thérapeutique antitumoral peut également être un dérivé d'acide folique, en particulier le méthotrexate (MTX) ou ses dérivés, tels que la lysine (e-y)-MTX ou la lysine (ε-α)-MTX telles que décrites par Fitzpatrick et al. (Anti-cancer Drug Design 10, pp. 1-9 et pp. 11-24 (1995)) ou l'aminoptérine, en particulier la lysine (ε-γ)-AMPT et la lysine (ε-α)-AMPT.

Le ligand (W-Z) est ensuite lié par sa fonction carboxylique à l'extrémité α-amino de l'agent thérapeutique.

### Dérivés d'alcaloïde vinca.

Les dérivés d'alcaloïdes vinca selon la présente invention sont notamment des dérivés de vinblastine, de vincristine, de vindesine et de navelbine.

### A. Liaison en position 3.

1. L'acide déacétylvincristine, qui est formé par l'addition de lysine au groupe amino-e pour obtenir une lysine (e)-dAcVCR. Ensuite, le ligand (W-Z) est lié par son extrémité carboxylique au groupe α-amino de la lysine (ε)-dAcVCR.
2. L'acide déacétylvincristine peut être également obtenu en ajoutant une diamine aliphatique à la vincristine (NH₂-alkyl-NH₂) de manière à obtenir un NH₂-alkyl-dAcVCR, le ligand (W-Z) étant ensuite lié par son extrémité carboxylique au groupe α-amino de la lysine (e)-dAcVCR.

Les dérivés de vinblastine (VBL) et de navelbine (5'-noranhydrovinblastine) peuvent être liés au ligand (W-Z) de la même manière que décrit précédemment pour la vincristine.

### B. Liaison en position 4 .

1. La V₄-hemiaspartate-vincristine est formée à partir de vincristine par liaison de l'acide aspartique au travers de son groupe γ-carboxylique au groupe hydroxyl en position 4 de la vincristine (V₄). Ensuite, le ligand (W-Z) est lié par son extrémité carboxylique au groupe α-amino de la V₄-hemiaspartate-vincristine.
2. La V₄-lysyl-vincristine est formée à partir de vincristine en liant la lysine par son groupe α-carboxylique au groupe hydroxyl en position 4 de la vincristine (V₄). Ensuite, le ligand (W-Z) est lié par son extrémité carboxylique au groupe α-amino de la V₄-lysyl-vincristine.
3. La V₄-lysyl-vincristine est formée à partir de vincristine en liant la lysine par son groupe α-carboxylique au groupe hydroxyl en position 4 de la vincristine (V₄). Ensuite, le ligand (W-Z) est lié par son extrémité carboxylique au groupe e-amino de la lysine de la V₄-lysyl-vincristine. Selon une alternative, le ligand (W-Z) peut être lié à la fois aux groupes α- et e-amino de la V₄-lysyl-vincristine.
4. La V₄-β-Alanyl-vincristine est formée à partir de vincristine en liant la β-Alanyl par son groupe carboxylique au groupe hydroxyl en position 4 de la vincristine (V4).Ensuite, le ligand (W-Z) est lié par son extrémité carboxylique au groupe amino de la β-Alanyl de la V₄-β-Alanyl-vincristine.

Des dérivés de vinblastine, de vindesine et de navelbine peuvent être liés au ligand (W-Z) de la même manière que décrit ci-dessus pour la vincristine.

### Dérivés de calichéamycine.

La N-Acetyl-diméthylhydrazide dérivée de calichéamycine est obtenue par réaction d'un hydrazide de thiol sur la calichéamycine.

Le ligand (W-Z) est ensuite lié par son extrémité carboxylique à la N-Acetyl-diméthylhydrazide dérivée de calichéamycine.

### Dérivés de mitoxanthrone.

Un β-Alanyl dérivé de mitoxanthrone est préparé par liaison de la fonction carboxylique de la β-Alanyl aux chaînes latérales hydroxyl du mitoxanthrone.

Une mono- ou une bisubstitution est ensuite possible. Le ligand (W-Z) est ensuite lié par son extrémité carboxylique au groupe amino de la β-Alanyl-mitoxanthrone.

### Dérivés de cytosine arabinoside (ARA-C).

Le ligand (W-Z) est lié par son extrémité carboxylique au groupe amino de la cytosine arabinoside.

### Dérivés d'adénosine arabinoside (Ara-A).

Le ligand (W-Z) est lié par son extrémité carboxylique au groupe amino de l'adénosine arabinoside.

### Dérivés de fludarabine phosphate.

Le ligand (W-Z) est lié par son extrémité carboxylique au groupe amino du fludarabine phosphate.

### Dérivés de melphalan.

Le ligand (W-Z) est lié par son extrémité carboxylique au groupe amino du melphalan.

### Dérivés de bléomycine.

Le ligand (W-Z) est lié par son extrémité carboxylique au groupe amino de la bléomycine, de la péplomycine ou de la liblomycine.

### Dérivés de mitomycine

Le ligand (W-Z) est lié par son extrémité carboxylique au groupe amino en position 7 de la mitomycine.

### Dérivés de L-canayanine.

Le ligand (W-Z) est lié par son extrémité carboxylique au groupe α-amino de la L-canavanine.

### Dérivés de taxoïdes.

Un dérivé β-Alanyl de taxol est préparé par liaison de la fonction carboxylique du β-Alanyl aux chaînes latérales hydroxyl en position 7 du taxol.

Le groupe hydroxyl du taxol est réactif mais pas essentiel à l'activité antitumorale du taxol (Nicalaou K.C. et al., Chemistry and Biology of Taxol, Angew. Chem. Int. Ed. Engl. (1994), 33, pp. 15-44).

Ensuite, le ligand (W-Z) est lié par son extrémité carboxylique au groupe amino du β-Alanyl-taxol.

Le ligand (W-Z) peut être lié de manière comparable au dérivé β-Alanyl-taxotère.

### Dérivés de camptothécine.

Le ligand (W-Z) est lié par son extrémité carboxylique au groupe α-amino de la 9-amino-camptothécine ou de la 7-amino-méthyl-camptothécine.

La présente invention sera décrite de manière plus détaillée dans les exemples suivants donnés à titre d'illustration non limitative de la présente invention.

### Exemple 1.

### Synthèse de la N-L-Leucyl-daunorubicine (Leu-DNR)

La L-Leucyl-daunorubicine est synthétisée par réaction de la daunorubicine sous forme de base (DNR) avec la L-Leucine protégée sur sa fonction amine par un groupe FMOC (fluorenyl-méthoxy-carbonyle) et dont la fonction carboxylique est activée par de l'IBCF (isobutylchloroformiate), puis déprotection de la fonction amine.

La DNR sous forme de base est préparée à partir de 200 mg de chlorhydrate de daunorubicine (R. Bellon) dissous dans 500 µl de DMF (dimethylformamide) auxquels on ajoute 1,2 équivalents de de N-Methyle-Morpholine.

1,2 équivalents de Fmoc-N-Leucine (NOVA BIOCHEM) sont dissous dans 500 µl de DMF (dimethylformamide), et 1,2 équivalents de N-Methyle-Morpholine et 1,2 équivalents d'IBCF sont ajoutés à -20°C. Après 15 minutes cette solution est ajoutée à celle de la DNR base et le mélange est laissé pendant 16 heures, sous agitation, à l'abri de la lumière.

La Fmoc-N-L-Leucyl-daunorubicine est précipitée dans 150 ml d'un mélange 1:1 d'éther et d'éther de pétrole (40-60°C) puis filtrée sur verre fritté n°4. Le produit est purifié par chromatographie sur colonne de silice (silice Si-60 70-23C mesh de E. MERCK) éluée par du chloroforme. La DNR résiduelle est éluée par 10% de Méthanol. Les fractions contenant la FMOC-N-L-Leucyl-DNR sont rotavapées, le produit repris dans 500 µl de DMF. La déprotection est réalisée en ajoutant 5 équivalents de diéthylamine (LAB SCAN) à -20°C.

Après 1 heure la N-L-Leucyl-DNR est précipitée par un mélange 1:1 d'éther et d'éther de pétrole (40-60°C), puis filtrée sur verre fritté no4. Le produit est repris dans un mélange chloroforme/méthanol (4:1 en volume) et la diéthylamine est neutralisée par un équivalent d' HCl.

Le produit est ensuite purifié par chromatographie sur colonne de silice (silice Si-60 70-230 mesh de E. MERCK) éluée par du chloroforme, puis du chloroforme contenant 15% de Méthanol. Les fractions contenant la N-L-Leucyl-DNR sont rotavapées, le produit repris dans de l'eau distillée et le chlorhydrate formé par ajustement du pH à 7 par de l' HCl 1N. Le produit est ensuite filtré sur gel de silice modifié (sep-pak C18 de WATERS), le chlorhydrate de N-L-Leucyl-daunorubicine est élué par du méthanol puis rotavapé. Le rendement habituel est de 70 à 80%.

### Caractéristiques des produits.

| **Composé** | **Formule** | **Point fusion** | **Rf TLC** | **Tr HPLC** |
|---|---|---|---|---|
| | | **(°C)** | | |
| DNR | C₂₇H₂₉NO₁₀, HCL | 189 | 0,05 | 0,67 |
| L-Leu-DNR | C₃₃H₄₀N₂O₁₁, HCL | 201 | 0,31 | 0,48 |

| | | | | |
|---|---|---|---|---|
| Rf TLC : système chloroforme : méthanol : eau (120:20:1, en volume) Tr HPLC : temps de rétention par rapport à la doxorubicine, système HPLC : colonne Si-60, éluée par un mélange chloroforme : méthanol : acide acétique : sol. aqueuse de MgCl2 0,3 mM (1440:420:80:60, en volume). | | | | |

### Synthèse de la β-L-Alanyl-L-Leucyl-L-Alanine.

La β-L-Alanyl-L-Leucyl-L-Alanine est préparée par synthèse en phase solide selon la technique de Merrifield (The Chemistry of Polypeptides, (P.G. Katsoyannis Ed.), Plenum Press, New-York, pp. 336-361 (1973)).

### Dérivés de fluorochromes.

Le ligand (W-Z) est lié par son extrémité carboxylique au groupement amino de la rhodamine 123.

### Synthèse de la β-Ala-Leu-Ala-Leu-DNR.

La β-Ala-Leu-Ala-Leu-DNR est synthétisée par greffage de la Fmoc-β-L-Alanyl-L-Leucyl-L-Alanine, préparée par synthèse en phase solide, sur la N-L-Leucyl-daunorubicine.

A la Fmoc-β-L-Alanyl-L-Leucyl-L-Alanine (1,5 équivalents) dissoute dans la DMF sont ajoutés 1,5 équivalents (eq.) de N-méthyle-morpholine et 1,5 eq. d'isobutyl chloroformiate. Après 10 minutes à -20°C, 1,5 eq. d'HOBT sont ajoutés. Puis après 5 minutes, 1 eq. de N-L-Leucyl-daunorubicine base dissous dans la DMF est ajouté. Après réaction, la Fmoc-β-Ala-Leu-Ala-Leu-DNR est précipitée à l'éther, puis déprotégée par la diéthyleamine. La β-Ala-Leu-Ala-Leu-DNR est purifiée par chromatographie sur colonne de silice et le chlorhydrate formé par addition d'HCl 1N.

### Synthèse de la β-Ala-Leu-Ala-Leu-DOX.

La β-Ala-Leu-Ala-Leu-DOX est synthétisée par greffage de la β-L-Alanyl-L-Leucyl-L-Alanine, préparée par synthèse en phase solide, sur la N-L-Leucyl-doxorubicine, tel que décrit pour la synthèse de la β-Ala-Leu-Ala-Leu-DNR.

Dans cette synthèse, l'agent de couplage (isobutyl chloroformiate) peut être omis de manière à augmenter encore le rendement de production du composé selon l'invention dans le cas de formation de β-Ala-Leu-Ala-Leu-DOX.

### Exemple 2.

### Dégradation de la β-Ala-Leu-Ala-Leu-DNR dans un milieu conditionné de cellules de carcinome mammaire MCF7/6.

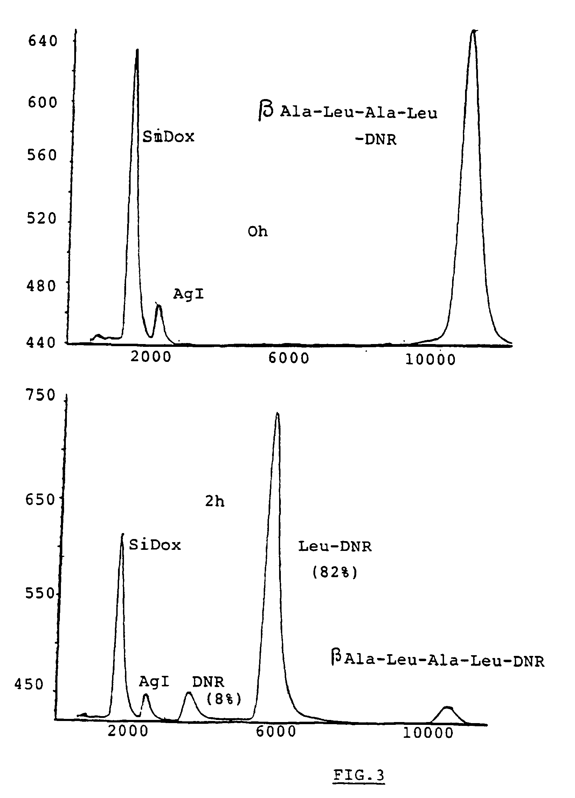

La β-Ala-Leu-Ala-Leu-DNR a été incubée à 37°C pendant 2 heures dans du milieu conditionné de cellules MCF7/6, concentré 20 ou 4 fois, et le composé ainsi que les produits de digestion ont été extraits à pH 9 et analysés par HPLC (figure 3).

| **Produit de départ ou Métabolite** | **Temps 0** | **Après 1 heure** | **Après 2 heures** |
|---|---|---|---|
| **β-Ala-L-Leu-L-Ala-L-Leu-DNR** | 100 % | 10 % | 2,6 % |
| L-Leu-L-Ala-L-Leu-DNR ou DNR | 0 % | 3,0 % | 7,3 % |
| L-Ala-L-Leu-DNR | 0 % | < 1 % | < 1 % |
| L-Leu-DNR | 0 % | 83 % | 90 % |

Il en ressort que la L-Leu-DNR est le métabolite majeur de l'endopeptidase et que sa formation ) partir de la prodrogue est quasi complète après 1 heure d'incubation à 37°C. Dans les conditions expérimentales utilisées, la L-Leu-L-Ala-L-Leu-DNR et la DNR ne sont pas suffisamment séparées pour que l'on puisse affirmer avec certitude que la L-Leu-DNR formée s'hydrolyse ensuite plus lentement en DNR.

### Exemple 3.

### Dégradation de la β-Ala-Leu-Ala-Leu-DOX dans le milieu conditionné de cellules de carcinome mammaire MCF7/6.

Quand la β-Ala-Leu-Ala-Leu-DOX est incubée à 37°C pendant 2 heures dans du milieu conditionné de cellules MCF7/6 concentré 20 fois, on ne retrouve plus comme métabolite que de la L-Leu-DOX et de la DOX.

| **Produit de départ ou Métabolite** | **Temps 0** | **Après 2 heures** |
|---|---|---|
| **β-Ala-L-Leu-L-Ala-L-Leu-DOX** | 100 % | 23 % |
| L-Leu-L-Ala-L-Leu-DOX | 0 % | < 1 % |
| L-Ala-L-Leu-DOX | 0 % | < 1 % |
| L-Leu-DOX | 0 % | 68 % |
| DOX | 0 % | 9 % |

La β-Ala-Leu-Ala-Leu-DOX est hydrolysée moins rapidement que la prodrogue de la DNR par la ou les peptidases secrétées par les cellules MCF7/6. Par contre, la transformation de la L-Leu-DOX en DOX semble un peu plus importante que la transformation de la L-Leu-DNR en DNR.

### Exemple 4.

### Dégradation de la β-Ala-Leu-Ala-Leu-DNR et de la β-Ala-Leu-Ala-Leu-DOX dans le sang humain.

Tout comme la β-Ala-Leu-Ala-Leu-DNR, la β-Ala-Leu-Ala-Leu-DOX est stable quand elle est incubée à 37°C dans du sang humain. En effet, moins de 2 % de la prodrogue est hydrolysée en L-Leu-DOX après 2 heures, tandis que les autres dérivés synthétisés s'hydrolysent rapidement en présence de sang humain (voir tableau 1 ci-dessous).

**Tableau 1 : HYDROLYSE DE DERIYES DE LA DAUNORUBICINE (DNR) ET DE LA DOXORUBICINE (DOX)**

| COMPOSE | Sang Humain | Milieu Conditionné MCF-7/6 | |
|---|---|---|---|
| L-Leu-DNR | + | - | Hydrolyse en DNR |
| D-Leu-DNR | - | - | " |
| diEthyle-β-Ala-DNR | - | - | " |
| L-NorLeu-DNR | - | - | |
| L-Ala-L-Leu-DNR | + | + | hydrolyse en L-Leu-DNR |
| L-Ala-L-Ala-DNR | + | - | Hydrolyse en DNR |
| L-Ala-L-Ileu-DNR | + | + | " |
| L-Ala-L-NorLeu-DNR | - | - | " |
| β-Ala-L-Leu-DNR | - | - | " |
| L-Phé-L-Leu-DNR | + | (±) | " |
| L-Ala-L-Ala-L-Leu-DNR | + | - | Hydrolyse en L-Leu-DNR |
| L-NLeu-L-Ala-L-Leu-DNR | + | + | " |
| L-Leu-L-Ala-L-Leu-DNR | + | + | " |
| L-Ala-L-Leu-L-Ala-L-Leu-DNR | + | + | " |
| L-Ala-L-Leu-Gly-L-Leu-DNR | + | (±) | Hydrolyse en DNR |
| Gly-L-Leu-Gly-L-Leu-DNR | + | + | " |
| Succ-L-Ala-L-Leu-DNR | - | - | Hydrolyse en L-Leu-DNR |
| Succ-L-Leu-L-Ala-L-Leu-DNR | - | - | " |
| Succ-L-Ala-L-Leu-L-Ala-L-Leu-DNR | (+) | - | " |
| pGlu-L-Ala-L-Leu-L-Ala-L-Leu-DOX | + | - | Hydrolyse en L-Leu-DOX |
| D-Leu-L-Ala-L-Leu-DNR | - | - | Hydrolyse en L-Leu-DNR |
| D-Ala-L-Leu-L-Ala-L-Leu-DNR | + | (+) | " |
| D-Leu-L-Ala-L-Leu-L-Ala-L-Leu-DNR | + | + | " |
| D-Leu-D-Ala-L-Leu-L-Ala-L-Leu-DNR | (+) | (+) | " |
| β-L-Ala-L-Leu-DNR | - | - | " |
| L-NLeu-β-L-Ala-L-Leu-DNR | + | + | " |
| β-Ala-L-Ala-L-Leu-DNR | - | - | " |
| β-Ala-L-Leu-L-Ala-L-Leu-DNR | - | + | Hydrolyse en L-Leu-DNR |
| β-Ala-L-Leu-L-Ala-L-Leu-DOX | - | + | Hydrolyse en L-Leu-DOX |
| β-Ala-L-Leu-L-Ala-L-Leu-COU* | - | + | Hydrolyse en L-Leu-COU |

### Exemple 5.

### Dégradation de la β-Ala-Leu-Ala-Leu-DNR dans le milieu conditionné de cellules de carcinome MCF7/ADR.

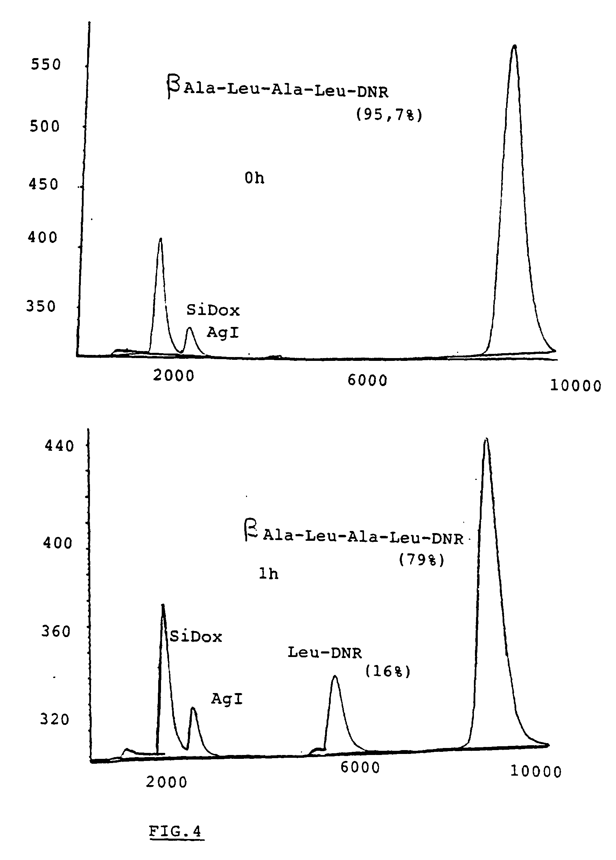

Quand la β-Ala-Leu-Ala-Leu-DNR est incubée à 37°C pendant 1 heure dans du milieu conditionné de cellules MCF7 résistantes aux anthracyclines (lignée MCF7/ADR), on ne retrouve comme métabolite que de la L-Leu-DNR (figure 4).

Les cellules MCF7/6, sensibles et résistantes aux anthracyclines, secrètent toutes deux la ou les protéases capables d'hydrolyser le composé selon l'invention.

### Exemple 6.

### Dégradation de la β-Ala-Leu-Ala-Leu-DNR dans le milieu conditionné de cellules d'hépatocarcinome HepG2.

Quand la β-Ala-Leu-Ala-Leu-DNR est incubée à 37°C pendant 2 heures dans du milieu conditionné de cellules d'hépatocarcinome HepG2, les métabolites principaux que l'on retrouve sont la L-Leu-DNR (27% de la fluorescence totale) et la L-Ala-L-Leu-DNR (8%). Le pourcentage d'hydrolyse ne peut être comparé entre les cellules de carcinome mammaire MCF7 et d'hépatocarcinome HepG2, car le nombre de cellules à partir desquelles les milieux conditionnés ont été récupérés, ainsi que les concentration des milieux conditionnés, sont différents. L'analyse qualitative indique néanmoins que la ou les peptidase(s) n'est (ne sont) pas spécifique(s) des cellules MCF7.

### Exemple 7.

### Dégradation de la β-Ala-Leu-A-Ala-Leu-DNR dans le milieu conditionné de cellules de carcinome du colon HT29.

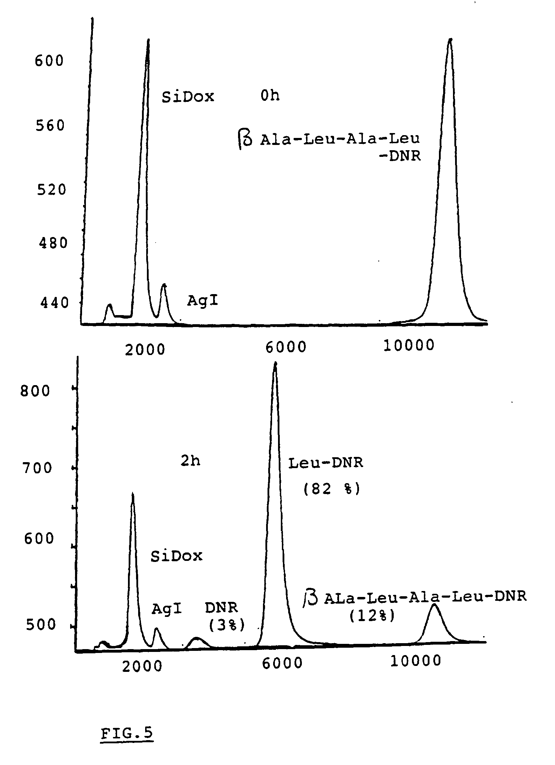

Quand la β-Ala-Leu-Ala-Leu-DNR est incubée à 37°C pendant 2 heures dans du milieu conditionné, concentré 40 fois, de cellules de carcinome du colon HT29, les métabolites principaux que l'on retrouve sont la L-Leu-DNR (82% de la fluorescence totale) et la L-Leu-L-Ala-L-Leu-DNR et/ou la DNR (3%), tel qu'illustré à la figure 5.

### Exemple 8.

### Accumulation sur cellules tumorales MCF7/6 de la DNR, de la L-Leu-DNR et de la β-Ala-L-Lau-L-Ala-L-Lau-DNR.

Les cellules du carcinome mammaire humain MCF7/6 ont été incubées en présence de β-Ala-Leu-Ala-Leu-DNR à une concentration de 10 µg eq. DNR/ml. Après divers temps, l'accumulation des prodrogues et de leurs métabolites fluorescents ont été déterminés par HPLC après extraction dans produits à pH basique, selon une méthode mise au point au laboratoire. Les accumulations, exprimés en µg/mg de protéines cellulaires, ont été comparées à celles de la DNR et de la DOX ainsi qu'à celles des L-Leu-DNR et L-Leu-DOX. Les métabolites ont été identifiés par détermination des temps de rétention de produits de référence synthétisés au laboratoire.

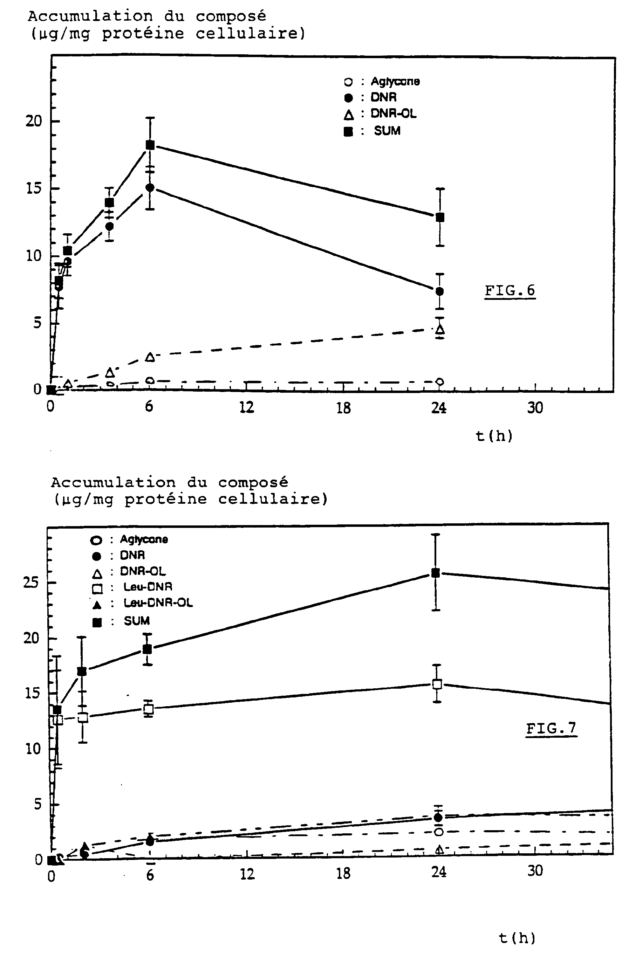

La DNR s'accumule rapidement dans les cellules MCF7/6 essentiellement sous forme inchangée, le maximum d'accumulation (± 15 µg/mg de protéines cellulaires) étant atteint après 6 heures d'incubation. L'accumulation de la DNR diminue ensuite jusqu'à 24 heures. Le métabolite principal intracellulaire est le daunorubicinol, résultant de la réduction de la fonction cétone de la DNR, en position C13, par des réductases intracellulaires (figure 6).

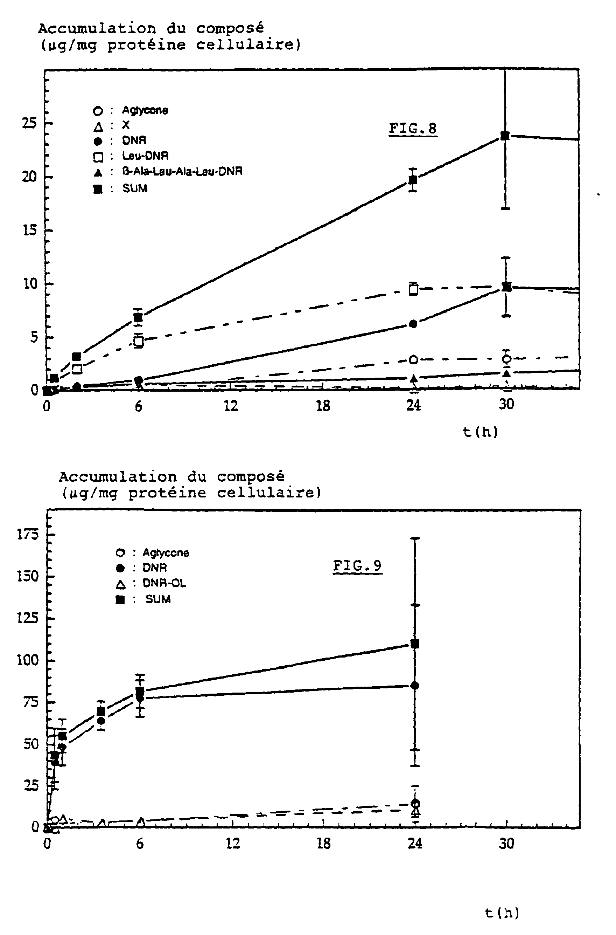

La L-Leu-DNR s'accumule également très rapidement par les cellules MCF7/6, mais à des taux moindres et l'accumulation atteint un plateau après 24 heures d'incubation, se situant à ± 14 µg/mg de protéines cellulaires. La DNR se forme intracellulairement de façon progressive au cours du temps pour atteindre 14% de la fluorescence totale intracellulaire après 24 heures d'incubation (figure 7).

La β-Ala-Leu-Ala-Leu-DNR, incubée pendant 24 heures en présence de cellules MCF7/6, s'accumule sous forme inchangée, beaucoup moins que la DNR et que la L-Leu-DNR, le taux d'accumulation n'étant que de ± 1 µg/mg de protéines cellulaires (figure 8). Ce sont essentiellement la L-Leu-DNR, formée extracellulairement, et la DNR que l'on retrouve dans les cellules après 24 heures d'incubation. La DNR retrouvée intracellulairement représente, après 24 heures d'incubation, environ 40% de la fluorescence totale.

**Tableau 2 : Concentrations intracellulaires en produit de départ L-Leu-DNR et DNR après 6 heures d'incubation des cellules MCF7/6 soit avec la DNR, la L-Leu-DNR ou la β-Ala-Leu-Ala-Leu-DNR.**

| **Incubation avec** | **Produit de départ** | **Leu-DNR formée** | **DNR formée** |
|---|---|---|---|
| | **(µg/mg protéines cellulaires)** | | |
| DNR | 14 , 9 | - | - |
| Leu-DNR | 13,4 | - | 1,5 |
| β-Ala-Leu-Ala-Leu-DNR | 0,3 | 4,5 | 0,9 |

Les résultats du tableau 1 indiquent que le passage des dérivés peptidiques de la DNR à travers les membranes cellulaires diminue quand la longueur de la chaîne peptidique augmente, et que la β-Ala-Leu-Ala-Leu-DNR est un précurseur de la Leu-DNR, activé extracellulairement. La Leu-DNR libérée s'accumule intracellulairement et devient elle-même un précurseur intracellulaire de la DNR.

### Exemple 9.

### Accumulation sur cellules normales MRC5 de la DNR, de la L-Leu-DNR et de la β-Ala-Leu-Ala-Leu-DNR.

Les cellules de fibroblastes MRC5 ont été incubées en présence de β-Ala-Leu-Ala-Leu-DNR à une concentration de 10 µg eq. DNR/ml. A divers temps, l'accumulation des prodrogues et de leurs métabolites fluorescents a été déterminée par HPLC après extraction des produits à pH basique, selon une méthode mise au point au laboratoire. Les accumulations, exprimés en µg/mg de protéines cellulaires, ont été comparées à celles de la DNR et de L-Leu-DNR. Les métabolites ont été identifiés par détermination des temps de rétention de produits de référence synthétisés au laboratoire.

La DNR s'accumule principalement dans les cellules MRC5 sous forme inchangée, et l'accumulation atteint un plateau de ± 76 µg/mg de protéines cellulaires après 6 heures, le métabolite majeur étant le daunorubicinol (figure 9).

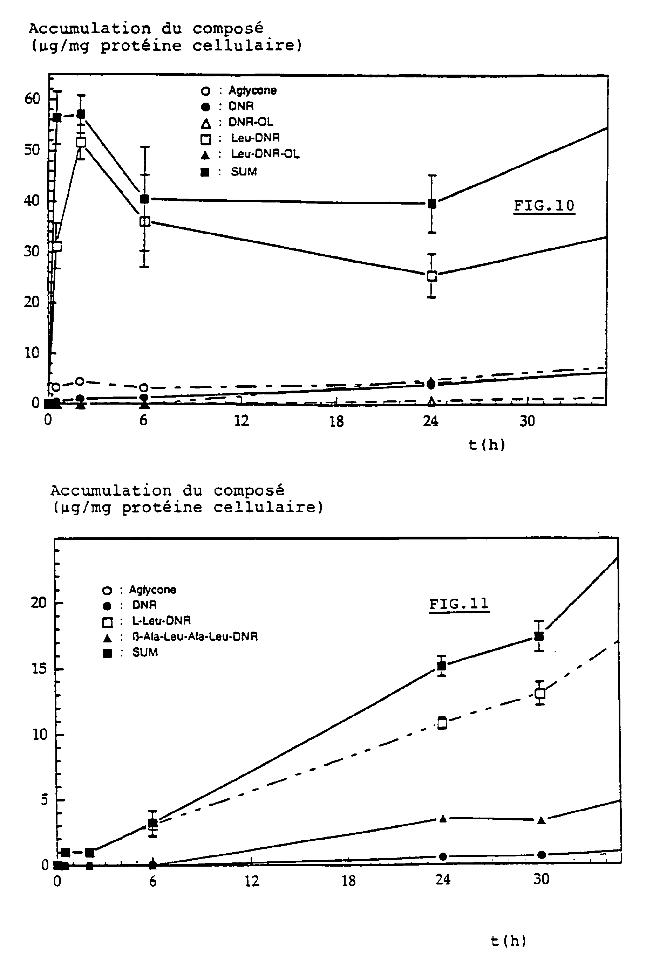

La L-Leu-DNR s'accumule également très rapidement par les cellules MRC5, mais à des taux moindres, et l'accumulation atteint un plateau après 24 heures d'incubation, se situant à ± 40 µg/mg de protéines cellulaires. Les métabolites principaux sont la DNR ainsi que le L-Leu-DNR-OL (figure 10).

La β-Ala-Leu-Ala-Leu-DNR, incubée pendant 24 heures en présence de cellules MRC5, s'accumule beaucoup moins que la DNR et que la L-Leu-DNR, le taux d'accumulation n'étant que de ± 3,3 µg/mg de protéines cellulaires (figure 11). C'est essentiellement la L-Leu-DNR, formée extracellulairement, que l'on retrouve dans les cellules. Son accumulation augmente linéairement jusqu'à 24 heures d'incubation.

Ces résultats corroborent ceux obtenus sur les cellules MCF7/6, à savoir que le composé β-Ala-Leu-Ala-Leu-DNR ne rentre pratiquement pas dans les cellules (=300 fois moins que la DNR après 6 heures) et que c'est la L-Leu-DNR formée extracellulairement qui s'accumule essentiellement dans les cellules MRC5 (tableau 3).

**Tableau 3 : Concentrations intracellulaires en produit de départ L-Leu-DNR et DNR après 6 heures d'incubation des cellules MRC5 soit avec la DNR, la L-Leu-DNR ou la β-Ala-Leu-Ala-Leu-DNR.**

| **Incubation avec** | **Produit de départ** | **Leu-DNR formée** | **DNR formée** |
|---|---|---|---|
| | **(µg/mg protéines cellulaires)** | | |
| DNR | 77,4 | - | - |
| Leu-DNR | 36,0 | - | 1,3 |
| β-Ala-Leu-Ala-Leu-DNR | 0,03 | 3,1 | 0,08 |

Dans la lignée tumorale MCF7/6, les taux intracellulaires obtenus de DNR après 24 heures d'incubation en présence du composé β-Ala-Leu-Ala-Leu-DNR sont 12 fois plus élevés que dans les fibroblastes MRC5.

Quand les cellules sont incubées en présence de Leu-DNR et de DNR, le rapport des taux intracellulaires de DNR n'est respectivement que de 0,37 et 0,19.

### Exemple 10.

### Cytotoxicité de la β-Ala-Leu-Ala-Leu-DNR vis-à-vis des cellules tumorales MCF7/6 et normales MRC5.

La cytotoxicité de la prodrogue DNR, de la Leu-DNR et de la DNR a été comparée sur cellules MCF7/6 et MRC5 maintenues en croissance pendant 72 heures en présence des divers composés.

Les cytotoxicités de la β-Ala-Leu-Ala-Leu-DNR, de la L-Leu-DNR et de la DNR ont été déterminées sur cellules MCF7/6 en croissance dans des boîtes à 96 puits, incubées en présence de concentrations croissantes des divers composés. Après 72 heures, les cellules sont incubées pendant 48 heures en absence d'anthracycline et la cytotoxicité est déterminée par mesure des protéines cellulaires par la technique de Bradford. Une gamme de 9 concentrations sont utilisées, allant de 700 µg/ml jusqu'à 0,0035 µg/ml, et chaque mesure représente une moyenne et une déviation standard de 6 valeurs. Les points expérimentaux sont ajustés à une courbe sigmoïde qui permet de calculer le point d'inflexion correspondant à la dose à laquelle la moitié des cellules survivent (IC₅₀).

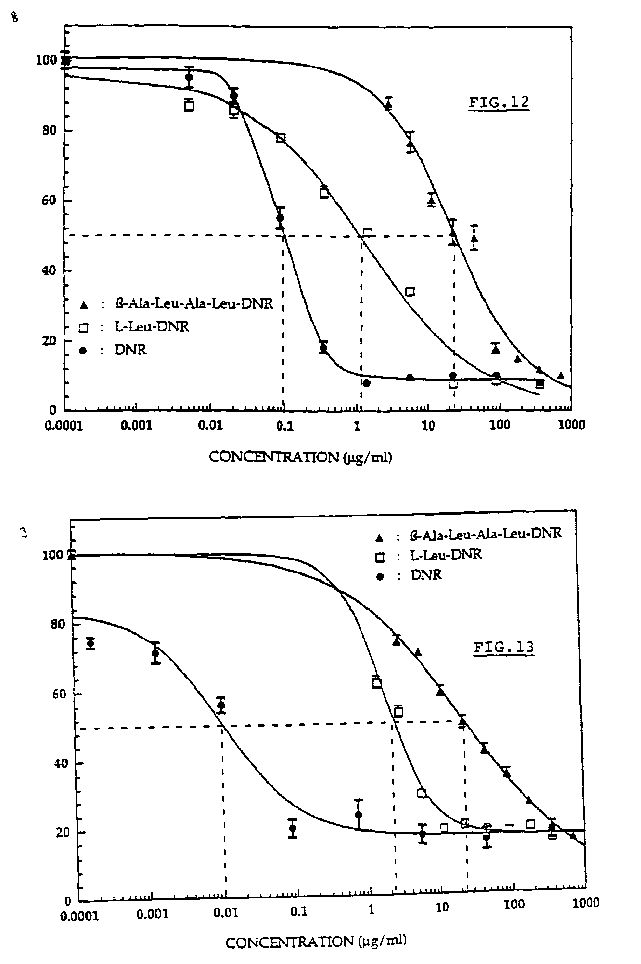

La figure 12 illustre que l'IC₅₀ de la DNR est de 0,090 ± 0,004 µg/ml. Pour les cellules incubées pendant 72 heures en présence de la L-Leu-DNR, l'IC₅₀ est de 1,30 ± 0,56 µg/ml. Dans le cas de la β-Ala-Leu-Ala-Leu-DNR, l'IC₅₀ est de 22,00 ± 7,31 µg/ml

La Leu-DNR et la β-Ala-Leu-Ala-Leu-DNR sont donc respectivement 14 fois et 244 fois moins cytotoxiques que la DNR pour les cellules du carcinome mammaire humain MCF7/6 maintenues en croissance pendant 72 heures en présence des anthracyclines.

Les cytotoxicités de la β-Ala-Leu-Ala-Leu-DNR, de la L-Leu-DNR et de la DNR ont été déterminées sur cellules MRC5 en croissance dans des boîtes à 96 puits, incubées en présence de concentrations croissantes des divers composés. Après 72 heures, les cellules sont incubées pendant 48 heures en absence d'anthracycline et la cytotoxicité est déterminée par mesure des protéines cellulaires par la technique de Bradford. Une gamme de 9 concentrations sont utilisées, allant de 700 µg/ml jusqu'à 0,0035 µg/ml, et chaque mesure représente une moyenne et une déviation standard de 6 valeurs. Les points expérimentaux sont ajustés à une courbe sigmoïde qui permet de calculer le point d'inflexion correspondant à la dose à laquelle la moitié des cellules survivent (IC₅₀).

La figure 13 illustre que l'IC₅₀ de la DNR est de 0,010 ± 0,006 µg/ml. Pour les cellules incubées pendant 72 heures en présence de la L-Leu-DNR, l'IC₅₀ est de 1,78 ± 0,23 µg/ml. Dans le cas de la β-Ala-Leu-Ala-Leu-DNR, l'IC₅₀ est de 23,14 ± 4,81 µg/ml.

La Leu-DNR et la β-Ala-Leu-Ala-Leu-DNR sont donc respectivement 172 fois et 2230 fois moins cytotoxiques que la DNR pour les cellules fibroblastiques MRC5 maintenues en croissance pendant 72 heures en présence des anthracyclines.

Aussi bien pour les cellules tumorales que pour les cellules fibroblastiques, le précurseur de la DNR est beaucoup moins toxique que le composé parent. Il reste à déterminer si cette diminution de toxicité observée in vitro s'observe également in vivo.

### Exemple 11.

### Toxicité aiguë in vivo.

La toxicité aiguë de la β-Ala-Leu-Ala-Leu-DNR a été comparée à celle de la daunorubicine sur souris. Dans l'étude in vivo de la toxicité aiguë d'un médicament, la détermination de la dose létale 50 (dose létale pour 50% des animaux ou LD₅₀) occupe une place importante. Malgré le fait qu'elle ne représente pas une constante biologique, elle donne des indications sur la toxicité aiguë du produit injecté. La LD₅₀ est un test simple où des quantités croissantes du produit à tester sont administrées par voie intraveineuse (i.v.) en une injection unique et par voie intrapéritonéale (i.p.) en 5 injections, une injection par jour pendant 5 jours consécutifs. La mortalité des animaux est suivie en fonction du temps. A la fin de la période d'observation, habituellement 30 jour, le valeur de la LD₅₀ est obtenue par régression linéaire du pourcentage de mortalité (sur échelle probit) en fonction du logarithme de la dose administrée (O. K. Chan and A. W. Hayes, Principles and methods for acute toxicity and eye irritancy, dans Principles and methods of toxicology, second edition. Ed. by A W. Hayes. Raven Press. New-York, USA (1989), pp. 169-220; D. Deprez-De Campeneere and A. Trouet. DNA-Anthracycline Complexes. I. Toxicity in mice and chemotherapeutic activity against L1210 Leukaemia of Daunorubicin-DNA and Adriamycin-DNA, Eur. J. Cancer, 16 (1980), pp. 981-986; H. E. Skipper, L. H. Schmidt, A. Goldin and J. M. Venditti. A manual on quantitative drug evaluation in experimental tumor systems, Cancer Chemother. Rep. 17 (1962), pp. 1-178).

Dans le cas où la LD₅₀ n'est pas atteinte, l'évolution des poids journaliers des souris donne également une indication sur la toxicité aiguë des produits administrés.

### 1. Matériel et méthodes.

La toxicité aiguë de ces deux drogues a été étudiée par les voies i.v. et i.p. sur une seule souche de souris. Les souris NMRI femelles (non consanguines IOPS-Han, d'environ 5 semaines et d'un poids moyen de 14,6 grammes, provenant d'IFFA-CREDO Belgique) sont restées en quarantaine pendant une semaine. A la veille du jour des injections, elles ont été distribuées en groupes de 5 (β-la-Leu-Ala-Leu-DNR) et de 7 (DNR) par dose à injecter; leur poids a été repris (environ 22 grammes en moyenne).

Les injections ont été effectuées systématiquement le matin (injection unique dans la veine caudale pour les i.v. et injections 5 jours consécutifs dans le péritoine pour les i.p.), en utilisant des seringues de 1.0 ml et des aiguilles 30G (i.v.) et 27G (i.p.) stériles. Toutes les manipulations d'animaux ont été effectuées avec des gants et l'entretien des animaux a été effectué systématiquement chaque semaine.

La β-Ala-Leu-Ala-Leu-DNR a été injectée par voie i.v. aux doses de 30, 60 et 120 mg/kg et par voie i.p. aux doses totales de 10, 15, 20, 25, 30, 45 et 60 mg/kg. Sur deux groupes supplémentaires, la β-Ala-Leu-Ala-Leu-DNR a été réinjectée par voie i.v. aux doses totales de 30 et 60 mg/kg en respectivement une et deux injections consécutives à 30 mg/kg.

La DNR a été injectée par voie i.v. aux doses de 10, 15, 20, 25, 30 et 35 mg/kg et par voie i.p. aux doses de 2,0; 2,5; 3,0 et 3,5 mg/kg.

Les solutions de β-Ala-Leu-Ala-Leu-DNR et de la DNR ont été réalisées dans du NaCl physiologique de telle sorte que le volume injecté corresponde à 0,1 ml pour 10 grammes de souris. Les concentrations des solutions ont été vérifiées par spectrophotométrie.

A partir du jour des injections (J0), les souris ont été suivies cliniquement, avec un relevé journalier des souris mortes. Le poids des souris a été mesuré presque tous les jours. La période d'observation a été prolongée au-delà du mois, afin de mieux repérer les signes de toxicité tardive. A la fin de l'étude, les souris survivantes ont été sacrifiés, selon les normes de l'expérimentation animale (D. B. McGregor. Ethics in experiments on animals, dans Experiments in toxicology, first edition. Ed. by D. Anderson and D. M. Conning. The Royal Society of Chemistry and The Universities Press. Belfast, Ireland (1988), pp. 512-522).

### 2. Résultants.

### Toxicité par voie intrapéritonéale.

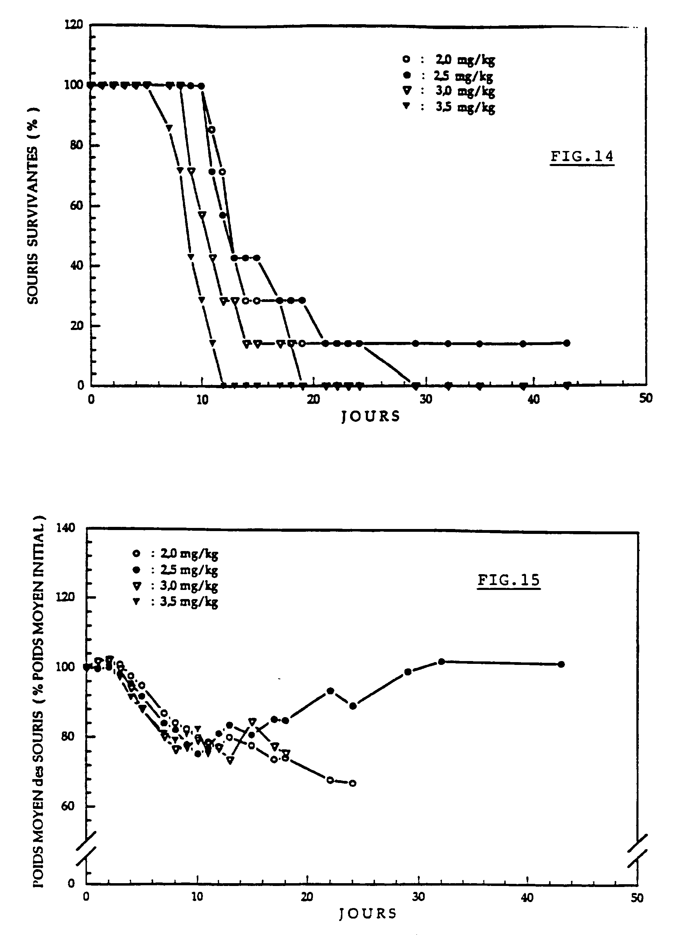

Tel qu'illustré à la figure 14, en ce qui concerne la DNR, une toxicité importante est observée en fonction de la concentration, toxicité qui se manifeste par la mortalité des souris, à partir du jour 7 à la dose de 3,5 mg/kg (1 souris morte sur 7), à partir du jour 9 à la dose de 3,0 mg/kg (2 souris mortes sur 7) et à partir du jour 11 aux doses de 2,5 et 2,0 mg/kg (respectivement 2 et 1 souris mortes sur 7).

Aucune souris ne survit au jour 12 (dose de 3,5 mg/kg), au jour 19 (dose de 3,0 mg/kg) et au jour 29 (dose de 2,0 mg/kg). La seule souris survivante au jour 43 est une de celles injectées à la dose de 2,5 mg/kg. Ces résultats confirment ceux décrits précédemment (D. Deprez-De Campeneere and A. Trouet. DNA-Anthracycline Complexes. I. Toxicity in mice and chemotherapeutic activity against L1210 Leukaemia of Daunorubicin-DNA and Adriamycin-DNA, Eur. J. Cancer, 16 (1980), pp. 981-986).

La figure 15 indique que quelle que soit la dose administrée, les souris accusent une perte de poids qui atteint 30% du poids initial. Cette perte de poids est généralement irréversible, sauf pour une souris traitée à 2,5 mg/kg, qui a récupéré son poids initial au jour 30.

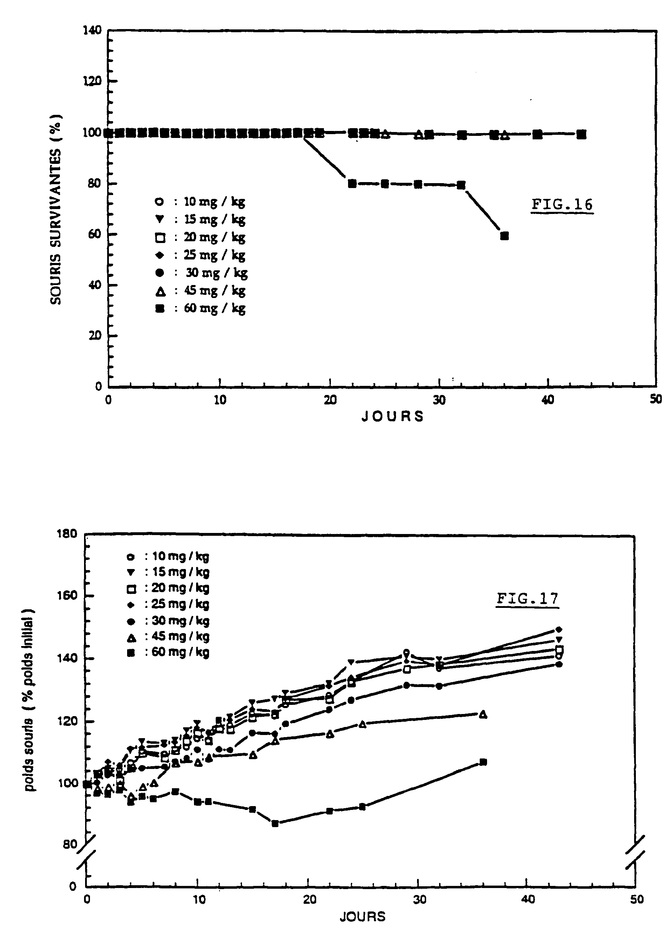

Quand la β-Ala-Leu-Ala-Leu-DNR est administrée par voie i.p. pendant 5 jours consécutifs à des doses totales comprises entre 10 et 45 mg/kg, aucune mortalité n'est observée. A la dose de 60 mg/kg, 2 souris meurent au jour 22 et une troisième au jour 35 (figure 16).

Aux doses totales comprises entre 10 et 45 mg/kg, aucune perte de poids n'est observée, mais plutôt un gain pondéral de ± 40% en 40 jours (figure 17). L'analyse des courbes de poids ne démontre pas de différence significative. Par contre, à la dose de 45 mg/kg, le poids moyen des souris est stable pendant les 7 premiers jours, puis l'augmentation de poids n'atteint que 20% au cours des 30 jours suivants. A la dose totale de 60 mg/kg, le poids moyen des souris diminue de ± 15% en 15 jours. Les deux souris survivantes ne récupèrent leur poids initial qu'au jour 36 (figure 17).

Ces données indiquent que la dose de 60 mg/kg de β-Ala-Leu-Ala-Leu-DNR est proche de la LD₅₀, et que donc, la β-Ala-Leu-Ala-Leu-DNR est au moins 30 fois moins toxique que la DNR, en terme de toxicité aiguë après administration i.p., 5 jours consécutifs.

### Toxicité par voie intraveineuse.

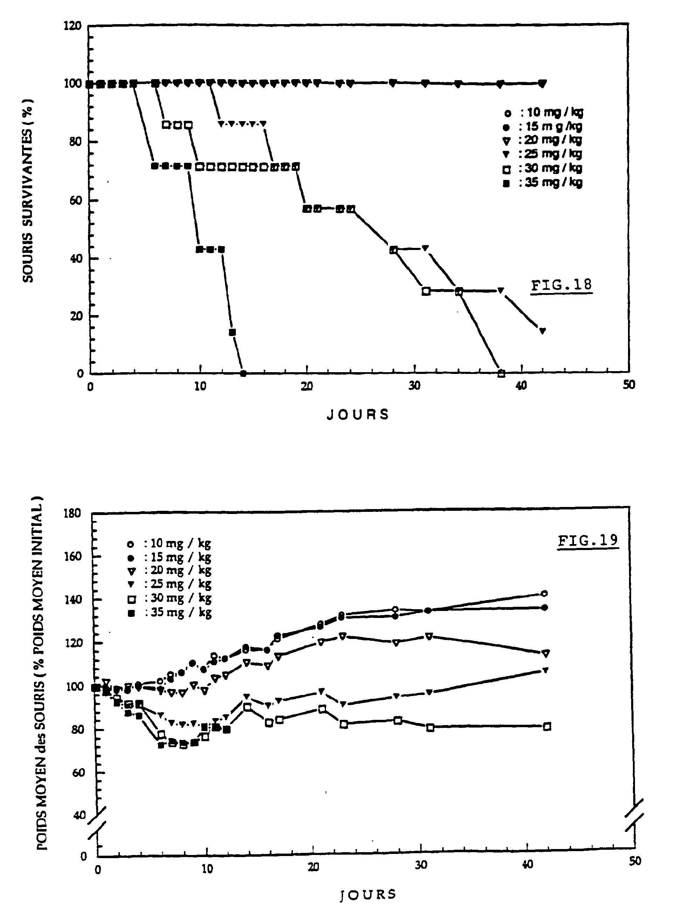

Tel qu'illustré à la figure 18, en ce qui concerne la DNR donnée par voie i.v. en administration unique à des souris NMRI femelles, aucune mortalité n'est observée aux faibles doses (10, 15 et 20 mg/kg). A 25 mg/kg, 6 souris meurent respectivement aux jours 12, 17, 20, 28, 34 et 42. Aux concentrations plus élevées de DNR, la mortalité commence le jour 7 (1 souris sur 7 à la dose de 30 mg/kg) et au jour 6 (2 souris sur 7 à la dose de 35 mg/kg). Ces résultats sont conformes à ceux décrits précédemment (D. Deprez-De Campeneere and A. Trouet. DNA-Anthracycline Complexes. I. Toxicity in mice and chemotherapeutic activity against L1210 Leukaemia of Daunorubicin-DNA and Adriamycin-DNA, Eur. J. Cancer, 16 (1980), pp. 981-986).

Les résultats de la figure 19 indiquent que la perte pondérale moyenne est maximale au jour 7 et est d'autant plus importante que la dose administrée est élevée. les souris survivantes aux doses de 30 et 35 mg/kg ne récupèrent pas leur poids initial 30 jours après l'injection i.v. de la DNR.

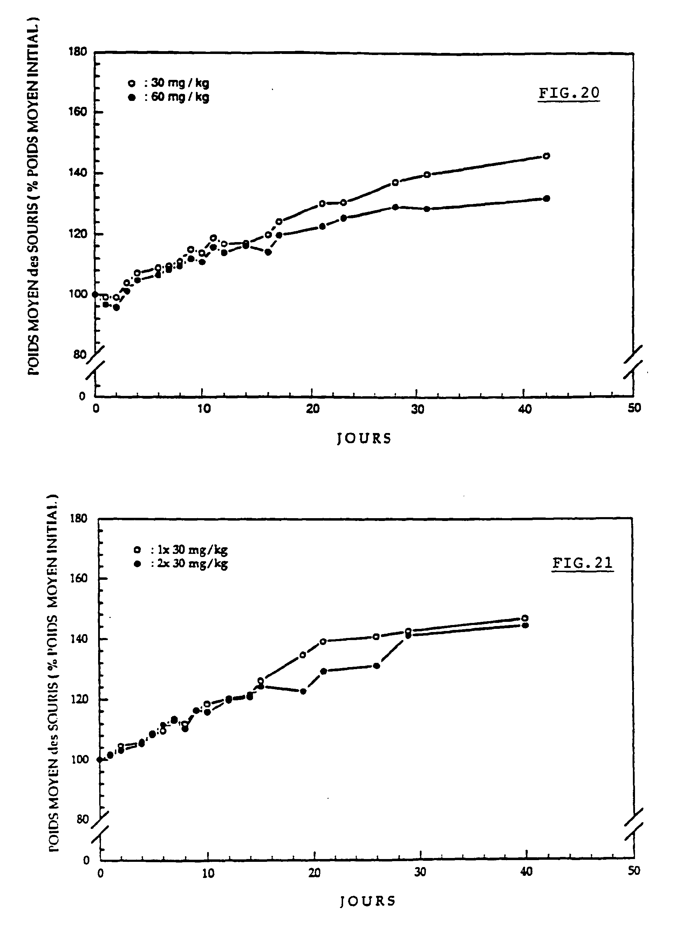

Quand la β-Ala-Leu-Ala-Leu-DNR est administrée pour voie i.v. à la dose de 30 mg/kg, aucune mortalité n'est observée, et le poids moyen des souris augmente de ± 40% en 40 jours (figure 20). Le poids moyen des souris à la fin de l'expérience est similaire à celui observé à la même dose de β-Ala-Leu-Ala-Leu-DNR administrée par voie i.p. Par contre, à la dose de 60 g/kg, 2 souris meurent endéans les 5 minute après l'injection. Cette mortalité peut être due à une hypervolémie suite à l'injection beaucoup trop rapide du produit. Les 3 souris survivantes, après un léger amaigrissement jusqu'au jour 2, récupèrent leur poids initial, puis augmentent de poids (± 30% en 40 jours). A la dose de 120 mg/kg, les 5 souris meurent endéans les 7 minutes suivant l'injection, avec des signes cliniques de toxicité.

Quand la β-Ala-Leu-Ala-Leu-DNR est administrée pour voie i.v. à la dose totale de 60 mg/kg, fractionnée en deux injections i.v. de 30 mg/kg chacune, deux jours consécutifs, aucune mortalité n'est observée. Il n'y a pas de différence dans l'évolution du poids moyen des souris ayant reçu 1 fois 30 mg/kg et celles ayant reçu 2 fois 30 mg/kg de β-Ala-Leu-Ala-Leu-DNR (figure 21).

### 3. Conclusions.

Les résultats obtenus permettent de conclure que tant par voie intraveineuse que par voie intrapéritonéale, la β-Ala-Leu-Ala-Leu-DNR a une toxicité aiguë beaucoup moins élevée que celle de la DNR, en terme de létalité. Ces résultats confirment la réduction de toxicité du dérivé β-Ala-Leu-Ala-Leu-DNR.

Des expériences identiques à celles décrites ci-dessus ont été réalisées avec des dérivés de doxorubicine.

### Exemple 12.

### Accumulation sur cellules tumorales MCF7/6 et cellules non tumorales MRC5 de la DOX, de la L-Leu-DOX et de la β-Ala-Leu-Ala-Leu-DOX.

Les cellules du carcinome mammaire humain MCF7/6 et de la lignée de fibroblastes humains MRC5 ont été incubées en présence de β-Ala-Leu-Ala-Leu-DOX à une concentration de 10 µg eq. DOX/ml. Après divers temps, l'accumulation des prodrogues et de leurs métabolites fluorescents a été déterminée par HPLC après extraction des produits à pH basique, selon une méthode mise au point au laboratoire. Les accumulations, exprimés en µg/mg de protéines cellulaires, ont été comparées à celles de la DOX et de L-Leu-DOX. Les métabolites ont été identifiés par détermination des temps de rétention de produits de référence synthétisés au laboratoire.

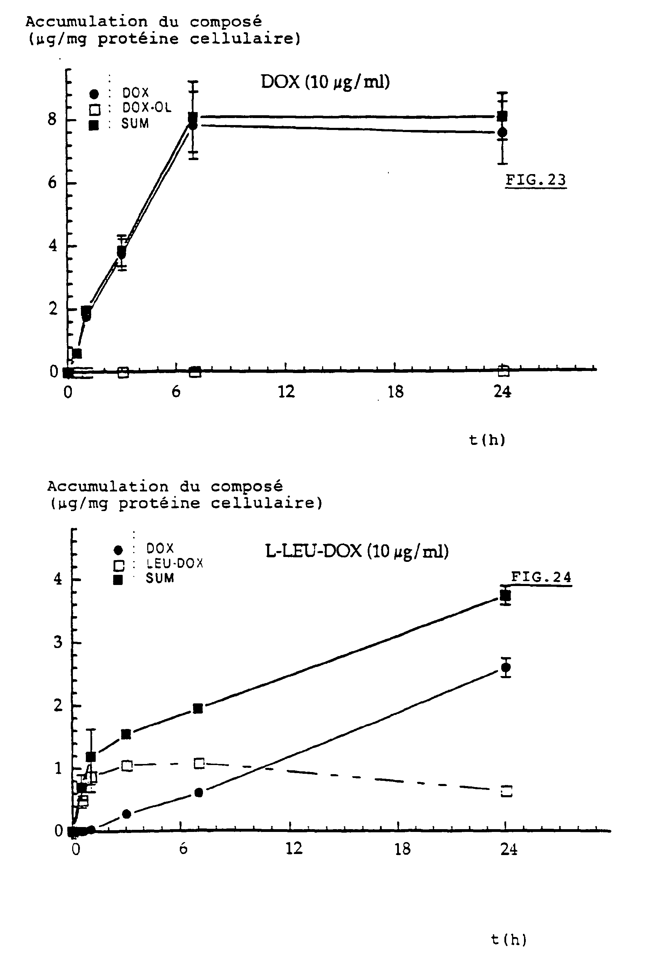

La DOX s'accumule principalement dans les cellules MCF7/6 essentiellement sous forme inchangée, et après 6 heures, un taux intracellulaire de 6,9 µg/mg de protéines cellulaires est atteint (figure 23).

| | | **Accumulation après 6 h** | | | **IC₅₀** |
|---|---|---|---|---|---|
| | | **(µg/mg prot. cellulaires)** | | | **(µg/ml)** |
| | | **βA-L-A-L-DOX** | **Leu-DOX** | **DOX** | **(72 h)** |
| **MCF7/6** | **DOX** | - | - | 6,90 | 0,0025 |
| | **Leu-DOX** | - | 1,10 | 0,25 | 0,02 |
| | **βA-L-A-L-DOX** | 0,10 | 0,65 | 0,22 | 3,0 |
| **MRC5** | **DOX** | - | - | 11,20 | 0,018 |
| | **Leu-DOX** | - | 1,40 | 0, 30 | 0, 3 |
| | **βA-L-A-L-DOX** | 0,10 | 0,10 | 0,01 | 120 |

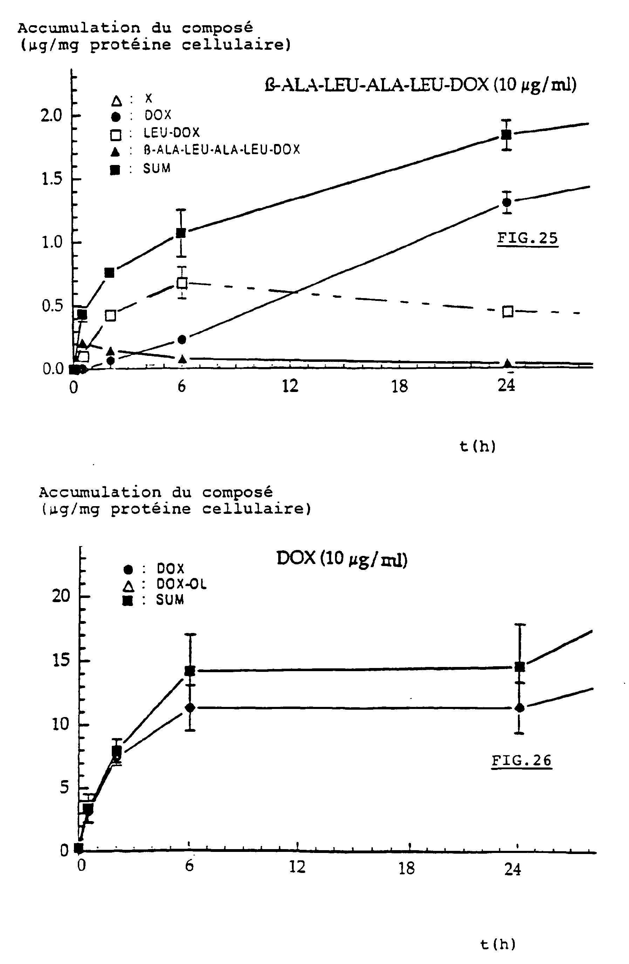

La Leu-DOX et la β-Ala-Leu-Ala-Leu-DOX s'accumulent respectivement 6 fois et 69 fois moins que la DOX après 6 heures. Intracellulairement, les taux de DOX sont 31 fois moins élevés après incubation des cellules en présence de β-Ala-Leu-Ala-Leu-DOX (figures 24 et 25).

Dans les cellules de fibroblastes MRC5, incubées pendant 6 heures en présence des anthracyclines à la concentration de 10 µg eq. DOX/ml, la DOX s'accumule essentiellement sous forme inchangée et l'accumulation atteint un taux de ± 11,2 µg/mg de protéines cellulaires après 6 heures (figure 26).

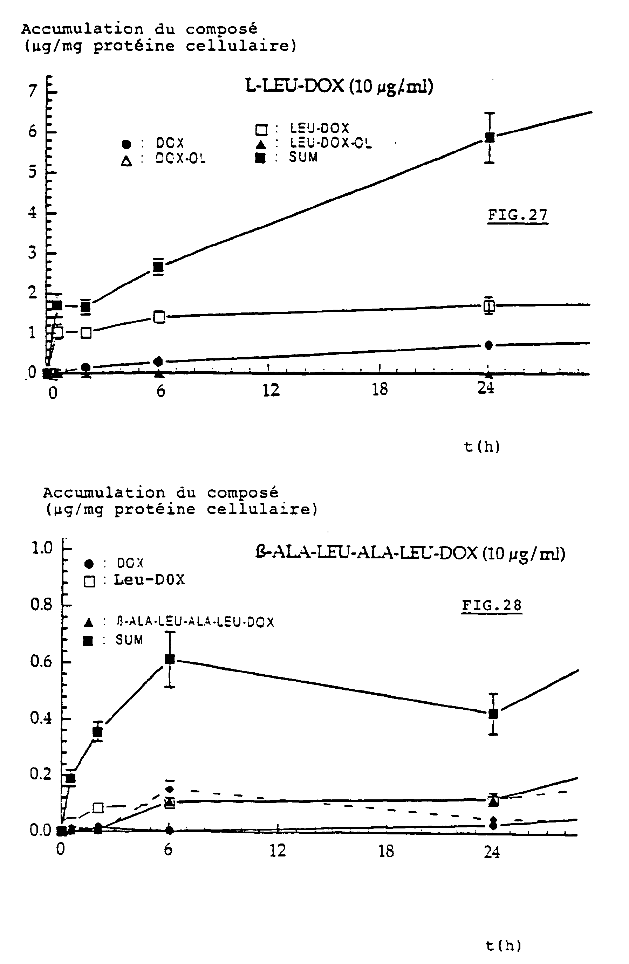

La L-Leu-DOX s'accumule à des taux moindres, l'accumulation atteignant 1,4 µg/mg de protéines cellulaires. Le métabolite principal est la DOX (0,3 µg/mg de protéines cellulaires) (figure 27).

La β-Ala-Leu-Ala-Leu-DOX s'accumule 112 fois moins que la DOX après 6 heures. Intracellulairement, les taux de DOX sont 1100 fois moins élevés après incubation des cellules en présence de β-Ala-Leu-Ala-Leu-DOX (figure 28).

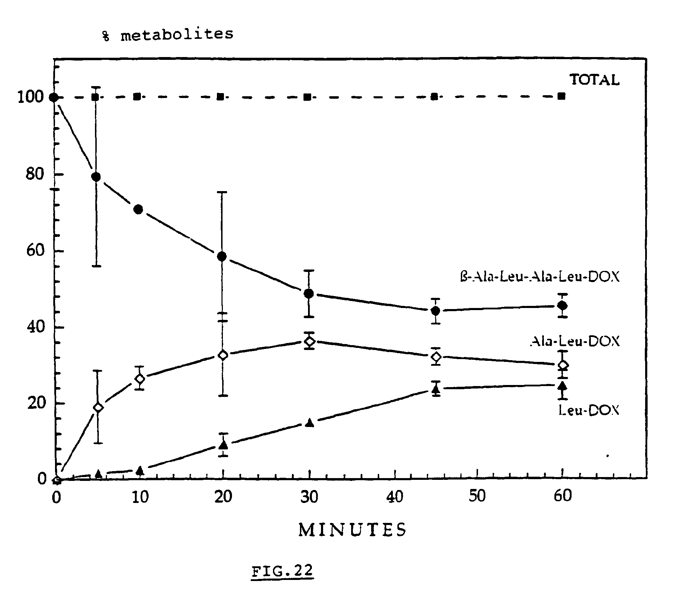

Ces résultats montrent qu'en tant que telle, la β-Ala-Leu-Ala-Leu-DOX ne rentre pratiquement pas dans les cellules, et qu'elle doit préalablement être hydrolysée dans le milieu extérieur sous forme de Leu-DOX avant de rentrer dans les cellules où intracellulairement la Leu-DOX peut ensuite générer la DOX (figure 22).

Ces résultats montrent également que les taux intracellulaires de l'agent thérapeutique activf sont deux fois plus élevés dans les cellules normales que dans les cellules tumorales dans le cas de la DOX. Par contre, dans le cas de la β-Ala-Leu-Ala-Leu-DOX, les taux intracellulaires de DOX sont 22 fois plus élevés dans les cellules tumorales MCF7 comparativement aux cellules non tumorales MRC5.

### Exempte 13.

### Cytotoxicité in vitro vis-à-vis des cellules tumorales MCF7/6 et des cellules non tumorales MRC5.

Les cytotoxicités de la β-Ala-Leu-Ala-Leu-DOX, de la L-Leu-DOX et de la DOX ont été déterminées sur cellules MCF7/6 et MRC5 en croissance dans des boîtes à 96 puits, incubées en présence de concentrations croissantes des divers composés. Après 72 heures, les cellules sont incubées pendant 48 heures en absence d'anthracycline et la cytotoxicité est déterminée par mesure des protéines cellulaires par la technique de Bradford. Une gamme de 9 concentrations sont utilisées, allant de 700 µg/ml jusqu'à 0,0035 µg/ml, et chaque mesure représente une moyenne et une déviation standard de 6 valeurs. Les points expérimentaux sont ajustés à une courbe sigmoïde qui permet de calculer le point d'inflexion correspondant à la dose à laquelle la moitié des cellules survivent (IC₅₀).

Le tableau de l'exemple 12 reprend les IC₅₀ qui sont respectivement de 0,0025, 0,020 et 3,0 µg/ml respectivement pour la DOX, la L-Leu-DOX et la β-Ala-Leu-Ala-Leu-DOX pour les cellules du carcinome mammaire humain MCF7/6. Pour les cellules de la lignée de fibroblastes humains MRC5, les valeurs sont respectivement de 0,018, 0,30 et 120 µg/ml.

Ces résultats indiquent que la Leu-DOX et la β-Ala-Leu-Ala-Leu-DOX sont respectivement 8 fois et 1000 fois moins cytotoxiques que la DOX pour les cellules du carcinome mammaire humain MCF7/6 fibroblastiques maintenues en croissance pendant 72 heures en présence des anthracyclines. En ce qui concerne les cellules MRC5, la Leu-DOX et la β-Ala-Leu-Ala-Leu-DOX sont respectivement 17 fois et 6700 fois moins cytotoxiques que la DOX pour les cellules maintenues en croissance pendant 72 heures en présence des anthracyclines.

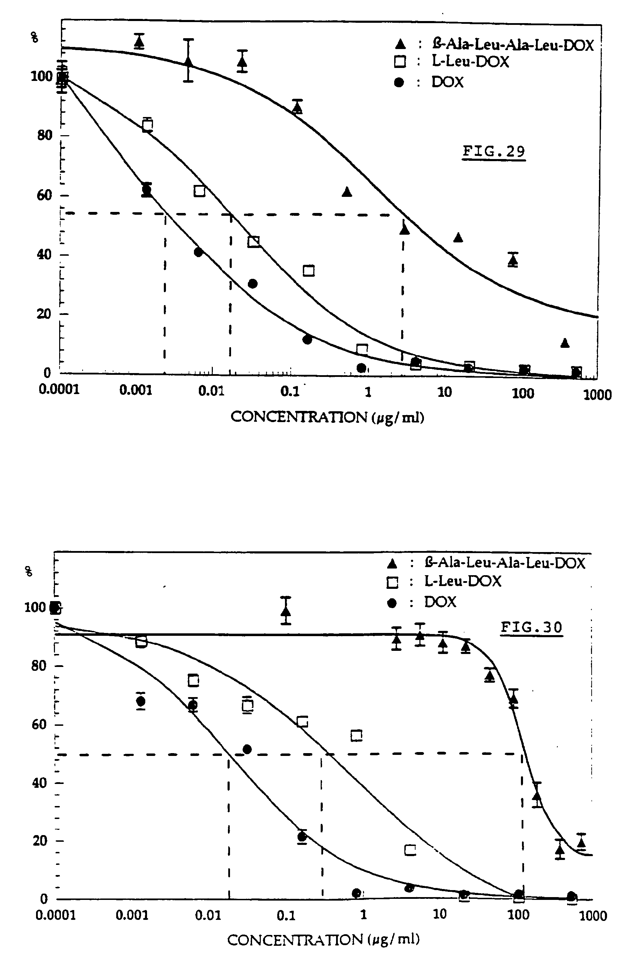

La β-Ala-Leu-Ala-Leu-DOX est 40 fois plus toxique pour les cellules tumorales MCF7 que pour les cellules non tumorales MRC5. Ceci est à mettre en relation avec les taux intracellulaires plus importants de DOX qui ont été observés dans les cellules MCF7 incubées en présence de β-Ala-Leu-Ala-Leu-DOX (figures 29 et 30).

Le composé est généralement caractérisé par une plus grande activité sur les modèles de tumeurs solides (par exemple par injection des cellules tumorales par voie sous-cutanée) que sur les modèles de type "leucémique" obtenus après injection intraveineuse des cellules tumorales.

Les cellules tumorales injectées par voie sous-cutanée forment une tumeur solide à l'endroit de l'injection et la concentration locale des hydrolases sécrétées par ces cellules restera importante. Quand les cellules tumorales sont injectées par voie intraveineuse, les hydrolases qu'elles sécrètent seront immédiatement diluées dans le flux sanguin.

### Exemple 14.

### Toxicité aiguë in vivo.

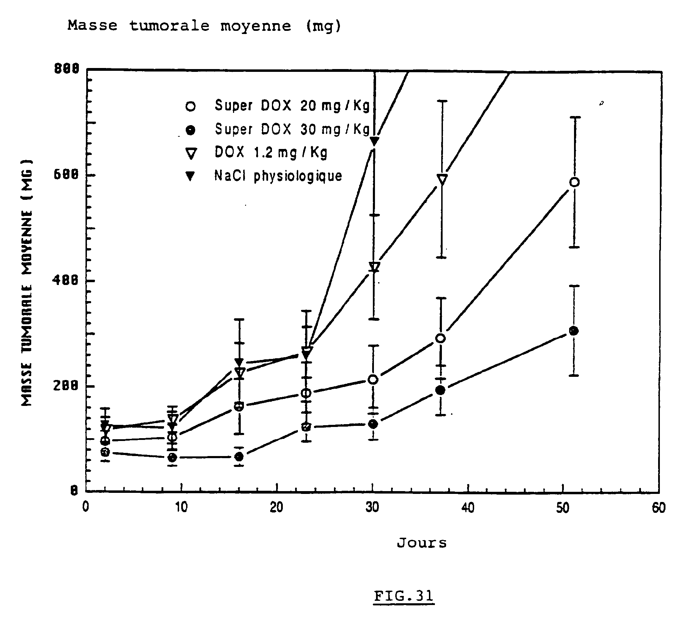

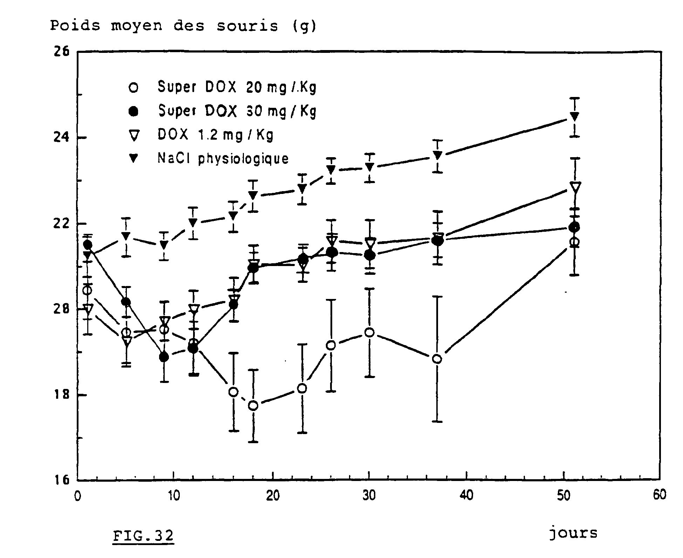

En outre, l'administration de la β-Ala-Leu-Ala-Leu-DOX à des souris athymiques auxquelles on a implanté une tumeur mammaire humaine MCF7/6 réduit l'évolution de la tumeur cancéreuse (figure 31) sans affecter de manière importante le poids moyen des souris traitées (figure 32).

Ces expérimentations ont été faites selon les protocoles décrits dans l'exemple 11.

Les Inventeurs ont également caractérisé la (les) protéase(s) secrétée(s) dans le milieu extracellulaire de cellules de carcinome mammaire humain qui peut (peuvent) hydrolyser la β-Ala-Leu-Ala-Leu-DOX. Il se confirme que cette (ces) protéase(s) ne correspond(ent) à aucune protéase décrite jusqu'à présent.

En effet, l'enzyme nommé provisoirement "COUM" est une métalloprotéase inhibable par les chélateurs de métaux tels que l'EDTA et nécessite pour son activité l'ion cobalt. Son pH optimum se situe entre 7,5 et 8,0, ce qui exclut que ce soit une cathepsine.

Par chromatographie à haute performance et par électrophorèse en présence de sulfate de lauryle, plusieurs bandes de poids moléculaire plus élevés que 70 kD sont observées.

## Revendications

1. Composé (W-Z-M) comprenant un agent thérapeutique antitumoral (M) possédant un site actif intracellulaire (S.A.), lié à un ligand (W-Z), comprenant un bras (Z) lié à un groupement terminal (W), **caractérisé en ce que** la liaison entre le groupement terminal (W) et le bras (Z) ainsi que la liaison entre le bras (Z) et l'élément (M) sont des liaisons covalentes n'affectant pas les propriétés du composé (W-Z-M), **en ce que** le bras (Z) du ligand (W-Z) est un peptide constitué d'au moins deux acides aminés éventuellement substitués, **en ce que** le groupement (W) est la β-Alanine de formule : NH₂-CH₂-CH₂-COOH, et **en ce que** la liaison entre l'élément (M) et le bras (Z) du ligand (W-Z) est un lien peptidique.

2. Composé selon la revendication 1, **caractérisé en ce que** le bras (Z) du ligand (W-Z) est choisi parmi le groupe constitué par les séquences peptidiques suivantes: L-Leucyl-L-Alanyl-L-Leucyl-, L-Leucyl-L-Alanyl-, L-Alanyl-L-Leucyl-L-Phénylalanyl-, L-Alanyl-L-Leucyl-.

3. Composé selon l'une quelconque des revendications précédentes, **caractérisé en ce que** l'agent thérapeutique antitumoral (M) est choisi parmi le groupe constitué par les anthracyclines, les dérivés d'acide folique, les alcaloïdes vinca, la calichéamycine, le mitoxanthrone, les cytosine arabinosides (ARA-C) ou adénosine arabinosides (ARA-A), le fludarabine phosphate, le melphalan, la bléomycine, la mitomycine, la L-canavanine, les taxoïdes, la camptothécine et leurs dérivés, en particulier le TOPOTECAN® (hydrochlorure de 9-diméthylaminométhyle-10-hydroxy camptothécine), éventuellement liés à un acide aminé substitué ou non.

4. Composé selon la revendication 3, **caractérisé en ce que** l'agent thérapeutique (M) est la doxorubicine ou la daunorubicine, éventuellement liées à un acide aminé substitué ou non.

5. Composé selon la revendication 4, **caractérisé en ce que** l'agent thérapeutique (M) correspond à la formule générale : 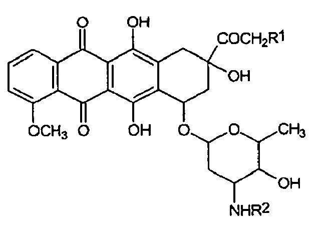 dans laquelle :
R¹ est un atome d'hydrogène ou un groupement OH,
R² est un atome d'hydrogène ou un radical de formule 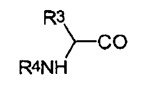
dans laquelle :
R³ est un atome d'hydrogène ou un radical alkyle, éventuellement substitué,
R⁴ représente un atome d'hydrogène ou forme avec R³ un radical alkylène comportant 3 ou 4 atomes de carbone.

6. Composé selon la revendication 5, **caractérisé en ce que** R² est un radical de formule 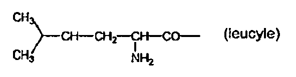 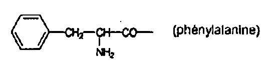 ou un de leurs isomères.

7. Composition pharmaceutique comprenant un composé (W-Z-M) comprenant un agent thérapeutique antitumoral (M) possédant un site actif intracellulaire (S.A.), lié à un ligand (W-Z), comprenant un bras (Z) lié à un groupement terminal (W), **caractérisé en ce que** la liaison entre le groupement terminal (W) et le bras (Z) ainsi que la liaison entre le bras (Z) et l'élément (M) sont des liaisons covalentes n'affectant pas les propriétés du composé (W-Z-M), **en ce que** le bras (Z) du ligand (W-Z) est un peptide constitué d'au moins deux acides aminés éventuellement substitués, **en ce que** le groupement (W) est la β-Alanine de formule : NH₂-CH₂-CH₂-COOH, et **en ce que** la liaison entre l'élément (M) et le bras (Z) du ligand (W-Z) est un lien peptidique, et éventuellement un adjuvant ou véhicule pharmaceutique acceptable.

8. Composition pharmaceutique selon la revendication 7, pour utilisation en tant que médicament.

9. Utilisation de la composition pharmaceutique selon l'une quelconque des revendications 7 ou 8, pour la préparation d'un médicament destiné au traitement des tumeurs cancéreuses.

## Claims

1. Compound (W-Z-M) comprising an antitumor therapeutic agent (M) having an intracellular active site (S.A.), bonded to a ligand (W-Z), comprising an arm (Z) bonded to a terminal grouping (W), **characterized in that** the bond between the terminal grouping (W) and the arm (Z), as well as the bond between the arm (Z) and the element (M), are covalent bonds not affecting the properties of the compound (W-Z-M), **in that** the arm (Z) of the ligand (W-Z) is a peptide comprised of at least two optionally substituted amino acids, **in that** the grouping (W) is β-alanine of the formula: NH₂-CH₂-CH₂-COOH, and **in that** the bond between the element (M) and the arm (Z) of the ligand (W-Z) is a peptide bond.

2. Compound according to claim 1, **characterized in that** the arm (Z) of the ligand (W-Z) is chosen from among the group comprised of the following peptide sequences: L-leucyl-L-alanyl-L-leucyl-, L-leucyl-L-alanyl-, L-alanyl-L-leucyl-L-phenylalanyl-, L-alanyl-L-leucyl-.

3. Compound according to any one of the previous claims, **characterized in that** the antitumor therapeutic agent (M) is chosen from among the group comprised of anthracyclines, folic acid derivatives, vinca alkaloids, calicheamicin, mitoxantrone, cytosine arabinosides (ARA-C) or adenosine arabinosides (ARA-A), fludarabine phosphate, melphalan, bleomycin, mitomycin, L-canavanine, taxoids, camptothecin and their derivatives, in particular TOPOTECAN® (9-dimethyl aminomethyl-10-hydroxy camptothecin hydrochloride), optionally bonded to a substituted or unsubstituted amino acid.

4. Compound according to claim 3, **characterized in that** the therapeutic agent (M) is doxorubicin or daunorubicin, optionally bonded to a substituted or unsubstituted amino acid.

5. Compound according to claim 4, **characterized in that** the therapeutic agent (M) corresponds to the general formula: 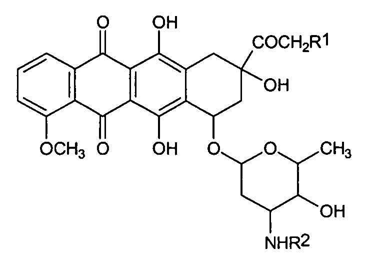 in which:
R¹ is an hydrogen atom or an OH grouping,
R² is an hydrogen atom or a radical of formula 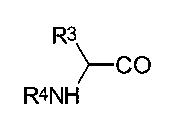
in which:
R³ is an hydrogen atom or an alkyl radical, optionally substituted,
R⁴ represents an hydrogen atom or forms with R³ an alkylene radical comprising 3 or 4 carbon atoms.

6. Compound according to claim 5, **characterized in that** R² is a radical of formula 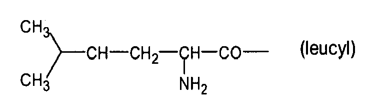 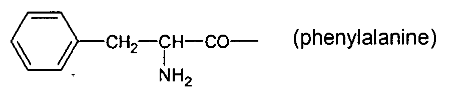 or one of their isomers.

7. Pharmaceutical composition comprising a compound (W-Z-M) comprising an antitumor therapeutic agent (M) having an intracellular active site (S.A.), bonded to a ligand (W-Z), comprising an arm (Z) bonded to a terminal grouping (W), **characterized in that** the bond between the terminal grouping (W) and the arm (Z), as well as the bond between the arm (Z) and the element (M), are covalent bonds not affecting the properties of the compound (W-Z-M), **in that** the arm (Z) of the ligand (W-Z) is a peptide comprised of at least two optionally substituted amino acids, **in that** the group (W) is β-alanine of formula: NH₂-CH₂-CH₂-COOH, and **in that** the bond between the element (M) and the arm (Z) of the ligand (W-Z) is a peptide bond, and optionally a pharmaceutically acceptable adjuvant or vehicle.

8. Pharmaceutical composition according to claim 7, for use as a medicament.

9. Use of the pharmaceutical composition according to any one of claims 7 or 8, intended for the manufacture of a medicament for the treatment of cancerous tumors.

## Patentansprüche

1. Verbindung (W-Z-M), die einen antitumoralen therapeutischen Wirkstoff (M) umfasst, der eine aktive intrazelluläre Stelle (S.A.) besitzt, die an einen Liganden (W-Z) gebunden ist, der einen Arm (Z) umfasst, der an eine Endgruppe (W) gebunden ist, **dadurch gekennzeichnet, dass** die Verbindung zwischen der Endgruppe (W) und dem Arm (Z) sowie die Verbindung zwischen dem Arm (Z) und dem Element (M) kovalente Bindungen sind, die die Eigenschaften der Verbindung (W-Z-M) nicht beeinträchtigen, **dadurch dass** der Arm (Z) des Liganden (W-Z) ein Peptid ist, das aus mindestens zwei gegebenenfalls substituierten Aminosäuren besteht, **dadurch, dass** die Gruppe (W) das β-Alanin der Formel: NH₂-CH₂-CH₂-COOH ist, und **dadurch, dass** die Verbindung zwischen dem Element (M) und dem Arm (Z) des Liganden (W-Z) eine peptidische Verbindung ist.

2. Verbindung nach Anspruch 1, **dadurch gekennzeichnet, dass** der Arm (Z) des Liganden (W-Z) ausgewählt ist aus der Gruppe, bestehend aus den folgenden peptidischen Sequenzen: L-Leucyl-L-Alanyl-L-Leucyl-, L-Leucyl-L-Alanyl-, L-Alanyl-L-Leucyl-L-Phenylalanyl-, L-Alanyl-L-Leucyl-.

3. Verbindung nach einem der vorhergehenden Ansprüche, **dadurch gekennzeichnet, dass** der antitumorale therapeutische Wirkstoff (M) ausgewählt ist aus der Gruppe bestehend aus Anthracyclinen, Folsäurederivaten, Vinca-Alkaloiden, Calicheamycinen, Mitoxanthronen, Arabinosylcytosinen (ARA-C) oder Arabinosyladenosinen (ARA-A), Fudarabinphosphat, Melphalan, Bleomycin, Mitomycin, L-Canavanin, Taxoiden, Camptothecin und dessen Derivaten, insbesondere TOPOTECAN® (9-Dimethylaminomethyl-10-hydroxycamptothecinhydrochlorid), das gegebenenfalls an eine substituierte oder nicht substituierte Aminosäure gebunden ist.

4. Verbindung nach Anspruch 3, **dadurch gekennzeichnet, dass** der therapeutische Wirkstoff (M) Doxorubicin oder Daunorubicin ist, die gegebenenfalls an eine substituierte oder nicht substituierte Aminosäure gebunden sind.

5. Verbindung nach Anspruch 4, **dadurch gekennzeichnet, dass** der therapeutische Wirkstoff (M) der allgemeinen Formel: 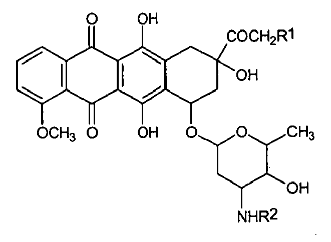 entspricht, worin:
R¹ ein Wasserstoffatom oder eine OH-Gruppe ist,
R² ein Wasserstoffatom oder ein Rest der Formel 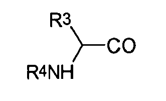 ist, worin:
R³ ein Wasserstoffatom oder ein gegebenenfalls substituierter Alkylrest ist,
R⁴ für ein Wasserstoffatom steht oder mit R³ einen Alkylenrest bildet, der 3 oder 4 Kohlenstoffatome umfasst.

6. Verbindung nach Anspruch 5, **dadurch gekennzeichnet, dass** R² ein Rest der Formel 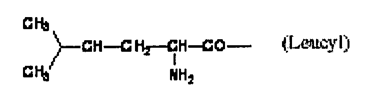 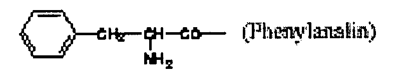 oder eines ihrer Isomere ist.

7. Pharmazeutische Zusammensetzung, die eine Verbindung (W-Z-M) umfasst, die einen antitumoralen therapeutischen Wirkstoff (M) umfasst, der eine aktive intrazelluläre Stelle (S.A.) besitzt, die an einen Liganden (W-Z) gebunden ist, der einen Arm (Z) umfasst, der an eine Endgruppe (W) gebunden ist, **dadurch gekennzeichnet, dass** die Verbindung zwischen der Endgruppe (W) und dem Arm (Z) sowie die Verbindung zwischen dem Arm (Z) und dem Element (M) kovalente Bindungen sind, die die Eigenschaften der Verbindung (W-Z-M) nicht beeinträchtigen, **dadurch dass** der Arm (Z) des Liganden (W-Z) ein Peptid ist, das aus mindestens zwei gegebenenfalls substituierten Aminosäuren besteht, **dadurch, dass** die Gruppe (W) das β-Alanin der Formel: NH₂-CH₂-CH₂-COOH ist, und **dadurch, dass** die Verbindung zwischen dem Element (M) und dem Arm (Z) des Liganden (W-Z) eine peptidische Verbindung ist, und gegebenenfalls ein Adjuvans oder pharmazeutisch unbedenkliches Vehikel.

8. Pharmazeutische Zusammensetzung nach Anspruch 7 zur Verwendung als Medikament.

9. Verwendung der pharmazeutischen Zusammensetzung nach einem der Ansprüche 7 oder 8 zur Herstellung eines Medikaments, das zur Behandlung von Krebstumoren bestimmt ist.
